# EUROPEAN PATENT APPLICATION

(11) **EP 4 850 697 A1**
(43) Date of publication of application: **09.12.2026**
(21) Application number: 25747784.4
(22) Date of filing: 24.01.2025
(51) Int. Cl.: A61K 47/54, C12N 15/113

(54) **LIPID OLIGONUCLEOTIDE CONJUGATE AND USE THEREOF**

(30) Priority: 30.01.2024 CN 202410129168
(71) Applicant: YUN HO BIO CO., LTD, Shanghai 201206 (CN)
(72) Inventor: WU, Yun, Shanghai 201206 (CN); ZHU, Yun, Shanghai 201206 (CN); MAO, Song, Shanghai 201206 (CN)
(74) Representative: Simmons & Simmons LLP (Munich)
(86) International application number: PCT/CN2025/074545
(87) International publication number: WO 2025/162216

(57) **Abstract**

A lipid oligonucleotide conjugate, an intermediate thereof, the use thereof and a pharmaceutical composition thereof. The lipid oligonucleotide conjugate can be used for the delivery of a variety of non-hepatic organs or tissue, including but not limited to muscles, fat, CNS, lungs, eyes, heart, etc., so as to achieve the treatment of diseases in the non-hepatic field.

## Description

### Technical Field

The present invention relates to delivery of oligonucleotides, in particular non-hepatic delivery of oligonucleotides. Specifically, the present invention relates to lipid-oligonucleotide conjugates, intermediates, uses, and pharmaceutical compositions thereof.

### Background

Nucleic acid drugs, unlike traditional small molecule drugs, possess advantages such as target specificity, broad therapeutic fields, and long-lasting efficacy, which endow them with unique clinical applications. By utilizing GalNAc ligands in combination with asialoglycoprotein receptor (ASGPR)-mediated endocytosis, efficient targeted delivery of nucleic acid drugs such as siRNA and ASO to the liver has been perfectly achieved. Currently, six GalNAc-nucleic acid drugs have been successfully marketed, and multiple drugs are in clinical stages. However, non-hepatic delivery of small nucleic acid drugs remains a major challenge in this field and limits further development of small nucleic acid drugs. Although attempts have been made with cholesterol-nucleic acid conjugates for non-hepatic delivery, their low delivery efficiency and toxicity caused by high-dose administration restrict clinical application of this approach.

Arrowhead disclosed a lipid structure designated LP300 in WO2023/245061, but with limited efficacy.

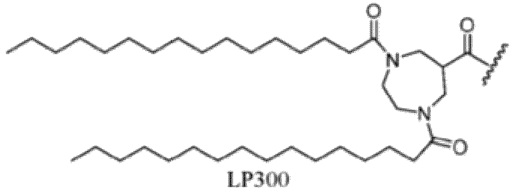

Therefore, designing and optimizing lipid structures to achieve non-hepatic delivery of lipidnucleic acid conjugates via lipid-assisted membrane permeation for treatment of diseases in non-hepatic fields is of significant clinical importance.

### Summary of Invention

The present invention is directed to providing lipid ligands for delivery of nucleic acid drugs, so as to achieve non-hepatic delivery of small nucleic acid drugs and expand the therapeutic fields of small nucleic acids for non-hepatic diseases. The lipid-oligonucleotide conjugates provided herein can be used for delivery to various non-hepatic organs or tissues, including but not limited to muscle, adipose tissue, CNS, lung, eye, heart, etc., thus enabling treatment of diseases in non-hepatic fields.

In one aspect, the present invention provides a lipid-oligonucleotide conjugate comprising a structure of Formula la: 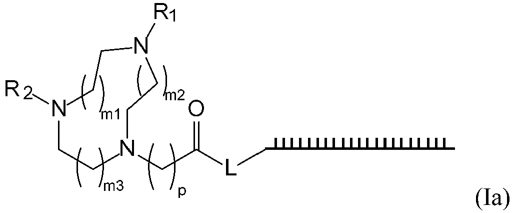 wherein,
at least one of R1 and R2 is selected from Ra, -C(O)Ra, -C(O)NHRa, and C(O)ORa, wherein Ra is optionally substituted C5-30 alkyl or alkenyl, and
the other of R1 and R2 is selected from H, Rb, -C(O)Rb, -C(O)NHRb, and C(O)ORb, wherein Rb is selected from optionally substituted C1-4 alkyl or alkenyl, optionally substituted C5-30 alkyl or alkenyl, optionally substituted C2-10 alkynyl, optionally substituted C1-10 alkoxy, optionally substituted C3-10 cycloalkyl, optionally substituted 6- to 14-membered aryl, optionally substituted 5- to 18-membered heteroaryl, optionally substituted 5- to 14-membered heterocyclyl;
said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5-to 18-membered heteroaryl, 5- to 14-membered heterocyclyl, halogen, hydroxyl, amino, guanidino, carboxyl, cyano, nitro, C1-3 alkylthio, and mercapto;
m1, m2, and m3 are each independently an integer of 1 or 2;
p is 0, 1, or 2; 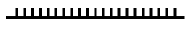
represents an oligonucleotide;
L represents a linker.

In another aspect, the present invention provides a lipid-oligonucleotide conjugate represented by Formula Ib, 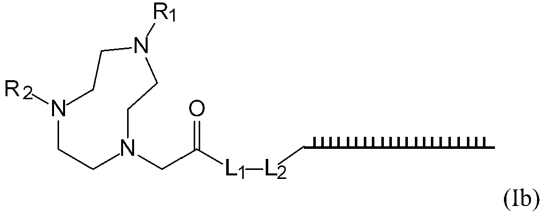 wherein,
at least one of R1 and R2 is selected from Ra, -C(O)Ra, -C(O)NHRa, and C(O)ORa, wherein Ra is optionally substituted C5-30 alkyl or alkenyl, and
the other of R1 and R2 is selected from H, Rb, -C(O)Rb, -C(O)NHRb, and C(O)ORb, wherein Rb is selected from optionally substituted C1-4 alkyl or alkenyl, optionally substituted C5-30 alkyl or alkenyl, optionally substituted C2-10 alkynyl, optionally substituted C1-10 alkoxy, optionally substituted C3-10 cycloalkyl, optionally substituted 6- to 14-membered aryl, optionally substituted 5- to 18-membered heteroaryl, optionally substituted 5- to 14-membered heterocyclyl;
said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5-to 18-membered heteroaryl, 5- to 14-membered heterocyclyl, halogen, hydroxyl, amino, guanidino, carboxyl, cyano, nitro, C1-3 alkylthio, and mercapto;
L1 is 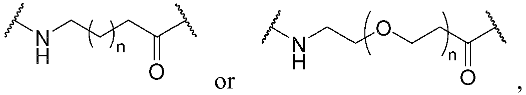 wherein the right side of the formula is connected to L2; L2 is 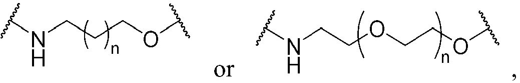 wherein the left side of the formula is connected to L1; the wavy line represents the connection position; and wherein each n is independently an integer from 1 to 10, preferably an integer from 2 to 8, more preferably an integer from 4 to 6; 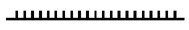
represents an oligonucleotide, preferably the oligonucleotide is an antisense oligonucleotide or the sense strand or antisense strand of an RNAi agent; preferably the 5' and/or 3' end of said oligonucleotide is linked to L2 via a phosphate group, phosphorothioate group, or phosphonate group; preferably the sense strand or antisense strand of said RNAi agent pairs with its antisense strand or sense strand to form a duplex; preferably said RNAi agent is siRNA; preferably said oligonucleotide is the sense strand of siRNA; preferably the antisense strand of said siRNA has a 5'-VP modification.

In another aspect, the present invention provides a lipid-oligonucleotide conjugate represented by Formula Ic, 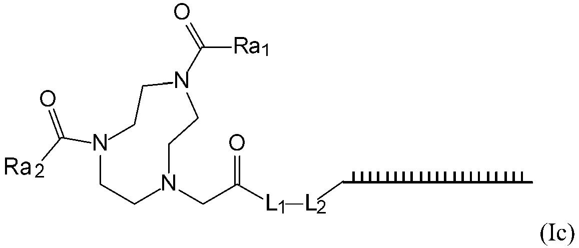 wherein,
Ra1 and Ra2 are each independently optionally substituted C5-30 alkyl or alkenyl, preferably optionally substituted C12-24 alkyl or alkenyl; preferably optionally substituted C14-22 alkyl or alkenyl; more preferably optionally substituted C14, C15, C16, C18, C20 or C22 alkyl, monounsaturated alkenyl, or polyunsaturated alkenyl; said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5- to 18-membered heteroaryl, 5- to 14-membered heterocyclyl, halogen, hydroxyl, amino, guanidino, carboxyl, cyano, nitro, C1-3 alkylthio, and mercapto; L1 is 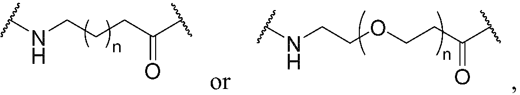 wherein the right side of the formula is connected to L2; L2 is 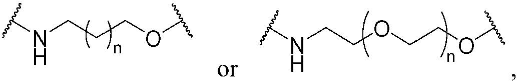 wherein the left side of the formula is connected to L1; the wavy line represents the connection position; and wherein each n is independently an integer from 1 to 10, preferably an integer from 2 to 8, more preferably an integer from 4 to 6; 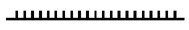
represents an oligonucleotide, preferably the oligonucleotide is an antisense oligonucleotide or the sense strand or antisense strand of an RNAi agent; preferably the 5' and/or 3' end of said oligonucleotide is linked to L2 via a phosphate group, phosphorothioate group, or phosphonate group; preferably the sense strand or antisense strand of said RNAi agent pairs with its antisense strand or sense strand to form a duplex; preferably said RNAi agent is siRNA; preferably said oligonucleotide is the sense strand of siRNA; preferably the antisense strand of said siRNA has a 5'-VP modification.

In another aspect, the present invention provides a ligand moiety represented by Formula II for forming a lipid-oligonucleotide conjugate: 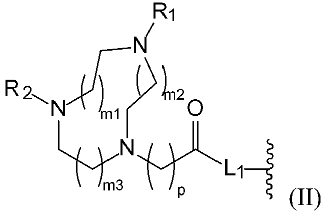 wherein,
at least one of R1 and R2 is selected from Ra, -C(O)Ra, -C(O)NHRa, and C(O)ORa, wherein Ra is optionally substituted C5-30 alkyl or alkenyl, and
the other of R1 and R2 is selected from H, Rb, -C(O)Rb, -C(O)NHRb, and C(O)ORb, wherein Rb is selected from optionally substituted C1-4 alkyl or alkenyl, optionally substituted C5-30 alkyl or alkenyl, optionally substituted C2-10 alkynyl, optionally substituted C1-10 alkoxy, optionally substituted C3-10 cycloalkyl, optionally substituted 6- to 14-membered aryl, optionally substituted 5- to 18-membered heteroaryl, optionally substituted 5- to 14-membered heterocyclyl;
said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5-to 18-membered heteroaryl, 5- to 14-membered heterocyclyl, halogen, hydroxyl, amino, guanidino, carboxyl, cyano, nitro, C1-3 alkylthio, and mercapto;
m1, m2, and m3 are each independently an integer of 1 or 2;
p is 0, 1, or 2; and 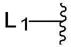
represents a linker, and the wavy line represents the position of attachment to the remainder of the lipid-oligonucleotide conjugate.

In another aspect, the present invention provides use of a ligand moiety represented by Formula III in the preparation of a lipid-oligonucleotide conjugate, 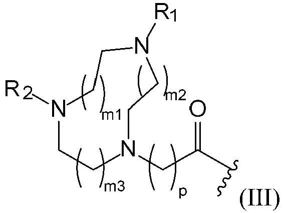 wherein,
at least one of R1 and R2 is selected from Ra, -C(O)Ra, -C(O)NHRa, and C(O)ORa, wherein Ra is optionally substituted C5-30 alkyl or alkenyl, and
the other of R1 and R2 is selected from H, Rb, -C(O)Rb, -C(O)NHRb, and C(O)ORb, wherein Rb is selected from optionally substituted C1-4 alkyl or alkenyl, optionally substituted C5-30 alkyl or alkenyl, optionally substituted C2-10 alkynyl, optionally substituted C1-10 alkoxy, optionally substituted C3-10 cycloalkyl, optionally substituted 6- to 14-membered aryl, optionally substituted 5- to 18-membered heteroaryl, optionally substituted 5- to 14-membered heterocyclyl;
said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5-to 18-membered heteroaryl, 5- to 14-membered heterocyclyl, halogen, hydroxyl, amino, guanidino, carboxyl, cyano, nitro, C1-3 alkylthio, and mercapto;
m1, m2, and m3 are each independently an integer of 1 or 2;
p is 0, 1, or 2; and
the wavy line represents the position of attachment to the remainder of the lipid-oligonucleotide conjugate.

Other aspects of the present invention will be apparent from the detailed description of the following specification.

### Brief Description of Figures

Figure 1. Inhibitory effects of lipid-siRNA conjugates **D1** and **D2** synthesized according to embodiments of the present invention on SOD1 mRNA expression in quadriceps muscle.
Figure 2. Inhibitory effects of lipid-siRNA conjugates **D1** and **D2** synthesized according to embodiments of the present invention on SOD1 mRNA expression in epididymal adipose tissue.

### Detailed Description of the Invention

### Definitions

As used herein, "oligonucleotide" refers to a sequence of nucleotides linked via internucleotide linkages to form a nucleotide chain, wherein each nucleoside and internucleotide linkage may be modified or unmodified. Unless otherwise indicated, an oligonucleotide consists of 12-30 linked nucleosides. As used herein, an oligonucleotide may be an "antisense oligonucleotide" or "ASO", the latter refers to a single-stranded oligonucleotide that can specifically bind to a target RNA (e.g., mRNA) sequence and modulate protein expression. In addition, an oligonucleotide may also be one strand of an RNAi agent, for example, the sense or antisense strand of siRNA, in which case the oligonucleotide preferably forms a complete RNAi agent with other parts of the RNAi agent. For example, when said oligonucleotide is the sense strand of siRNA, it is preferred that said oligonucleotide pairs with the antisense strand of siRNA to form a duplex (i.e., siRNA). Oligonucleotides include modified or unmodified oligonucleotides.

"Internucleotide linkage" means a covalent linkage between adjacent nucleotides in an oligonucleotide. As used herein, "modified internucleotide linkage" means any internucleotide linkage other than a phosphodiester internucleotide linkage. A "phosphorothioate internucleotide linkage" is a modified internucleotide linkage in which one of the non-bridging oxygen atoms of the phosphodiester internucleotide linkage is replaced by a sulfur atom.

"Deoxy region" means a region of 5-12 contiguous nucleotides, wherein at least 70% of the nucleosides are 2'-β-D-deoxynucleosides. In certain embodiments, each nucleoside is selected from 2'-β-D-deoxynucleosides, bicyclic nucleosides, and 2'-substituted nucleosides. In certain embodiments, the deoxy region supports RNase H activity. In certain embodiments, the deoxy region is the gap or internal region of a gapmer.

"Gapmer" means an antisense oligonucleotide comprising an internal region flanked by one or more nucleosides in external regions, having a plurality of nucleosides that support RNase H cleavage, wherein the nucleosides constituting the internal region are chemically different from the one or more nucleosides constituting the external regions. The internal region may be referred to as the "gap", and the external regions may be referred to as "wings". The internal region is a deoxy region. The position of the internal region or gap refers to the order of the nucleosides in the internal region and is counted from the 5' end of the internal region. Unless otherwise indicated, "gapmer" refers to a sugar motif. In certain embodiments, each nucleoside in the gap is a 2'-β-D-deoxynucleoside. In certain embodiments, the gap comprises one 2'-substituted nucleoside at position 1, 2, 3, 4, or 5 of the gap, and the remaining nucleosides of the gap are 2'-β-D-deoxynucleosides. As used herein, the term "MOE gapmer" indicates a gapmer having a gap comprising 2'-β-D-deoxynucleosides and wings comprising 2'-MOE nucleosides. As used herein, the term "mixed wing gapmer" refers to a gapmer having wings comprising modified nucleosides that contain at least two different sugar modifications. Unless otherwise indicated, gapmers may contain one or more modified internucleotide linkages and/or modified nucleobases, and such modifications do not necessarily follow the sugar-modified gapmer pattern.

As used herein, the term "RNAi agent" refers to an agent that comprises an RNA molecule capable of down-regulating expression of a target gene via an RNA interference mechanism when introduced into a cell. The term RNAi agent includes modified or unmodified RNAi agents. RNA interference refers to a process by which a nucleic acid molecule induces cleavage and degradation of a target RNA molecule (e.g., mRNA) in a sequence-specific manner, for example via the RNA-induced silencing complex (RISC) pathway. RNAi agents herein include siRNA, shRNA, and DNA/RNA hybrid molecules, sometimes collectively referred to as double-stranded RNA (dsRNA), which comprise two antiparallel contiguous polynucleotide strands that are sufficiently complementary to each other to hybridize and form a duplex region. "Hybridization" refers to the pairing of complementary polynucleotides, typically through hydrogen bonds (e.g., Watson-Crick hydrogen bonds, Wobble hydrogen bonds, Hoogsteen hydrogen bonds, or reverse Hoogsteen hydrogen bonds) between complementary bases in the two polynucleotides. "Duplex region" refers to a region in two complementary or substantially complementary polynucleotides that form base pairs by hybridization, thereby forming a duplex between the two polynucleotide strands.

The term "antisense strand" refers to the strand in a dsRNA that contains a region substantially complementary to a target sequence. The term "sense strand" or "passenger strand" refers to the strand in a dsRNA that contains a region substantially complementary to the antisense strand region as defined herein. The term "region substantially complementary" denotes a region that is either perfectly complementary or imperfectly complementary. When the complementary region is imperfectly complementary to the target sequence, mismatches may occur in internal or terminal regions of the molecule. Typically, the most tolerated mismatches are located in terminal regions, for example, within 5, 4, 3, or 2 nucleotides at the 5' and/or 3' termini of the dsRNA.

"siRNA" refers to a nucleic acid that forms a double-stranded RNA capable of reducing or inhibiting the expression of a target gene when present in the same cell as the target gene. An siRNA is typically approximately 15 to approximately 30 base pairs in length, most typically approximately 19 to 25 base pairs in length, for example, 19, 20, 21, 22, 23, 24, or 25 nucleotide pairs in length.

"shRNA" refers to short hairpin RNA, which comprises two short inverted repeats and an intervening stem-loop structure connecting them. The stem-loop may comprise at least one unpaired nucleotide, for example, at least 2, at least 3, at least 4, at least 5, at least 6, at least 7, at least 8, at least 9, at least 10, at least 20, at least 23, or more unpaired nucleotides. The stem-loop may be 10 or fewer nucleotides in length. The stem-loop may comprise 8 or fewer unpaired nucleotides. The stem-loop may comprise 4 to 10 unpaired nucleotides. The stem-loop may be 4 to 8 nucleotides in length.

The two substantially complementary strands of a dsRNA need not be, but may be, covalently linked. The maximum number of base pairs is the number of nucleotides in the shortest strand of the dsRNA minus any overhangs present in the duplex. In addition to the duplex structure, a dsRNA may comprise one or more nucleotide overhangs. An overhanging nucleotide refers to one or more unpaired nucleotides extending beyond the double-stranded region at the terminus of a strand. Nucleotide overhangs typically occur when the 3' end of one strand extends beyond the 5' end of the other strand, or when the 5' end of one strand extends beyond the 3' end of the other strand. For example, at least one strand comprises a 3' overhang of at least 1 nucleotide, e.g., 1 to 4 nucleotide overhangs. In another example, at least one strand comprises a 5' overhang of at least 1 nucleotide, e.g., 1 to 4 nucleotide overhangs. In other embodiments, both the 3' terminus and the 5' terminus of one strand of the dsRNA comprise an overhang of at least 1 nucleotide.

As used herein, the term "blunt end" or "blunt-ended" with respect to dsRNA means that there are no unpaired nucleotides or nucleotide analogs at a given end of the dsRNA, i.e., no nucleotide overhang. One or both ends of the dsRNA may be blunt. If both ends of the dsRNA are blunt, the dsRNA is referred to as blunt-ended. For clarity, a "blunt-ended" dsRNA is a dsRNA that is blunt at both ends, i.e., neither end of the molecule has a nucleotide overhang. Most often, such a molecule is double-stranded throughout its entire length. As used herein, the term "nucleotide overhang" refers to at least one unpaired nucleotide protruding from the duplex structure of the dsRNA. For example, a nucleotide overhang exists when the 3' end of one strand of the dsRNA extends beyond the 5' end of the other strand, or vice versa. Nucleotide overhangs may comprise or consist of nucleotides/nucleoside analogs, including deoxynucleotides/nucleosides. Overhangs may be on the sense strand, the antisense strand, or any combination thereof. Furthermore, overhang nucleotides may be present at the 5' end, the 3' end, or both ends of the antisense or sense strand of the dsRNA.

The dsRNA molecule may comprise chemical modifications to ribonucleotides, including modifications to the ribose, base, or backbone components of ribonucleic acid, as described herein or known in the art. Any such modifications, when used in double-stranded ribonucleic acid molecules (e.g., siRNA, shRNA, etc.), are encompassed by the term "dsRNA" for purposes of this disclosure. A "modified" nucleotide refers to a nucleotide that independently has a modified sugar moiety, a modified internucleoside linkage, and/or a modified nucleobase. Accordingly, the term "modified nucleotide" includes substitutions, additions, or removals of functional groups or atoms to the internucleoside linkage, sugar moiety, or nucleobase.

The term "alkyl" refers to a saturated straight or branched hydrocarbon chain. For alkyl groups having a specified number of carbon atoms, the term encompasses the corresponding normal alkyl and its various isomeric forms, if any. For example, an alkyl group having 4 carbon atoms (C4 alkyl) includes n-butyl, isobutyl, sec-butyl, and tert-butyl. Exemplary C1-10 alkyl groups include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, 4,4-dimethylpentyl, 5,5-dimethylhexyl, 6,6-dimethylheptyl, and the like.

The term "alkenyl" refers to a straight or branched hydrocarbon chain having one or more carbon-carbon double bonds. Exemplary C2-10 alkenyl groups include vinyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, and the like.

The term "alkynyl" refers to a straight or branched hydrocarbon chain having one or more carbon-carbon triple bonds. Exemplary C2-10 alkynyl groups include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 4-methyl-2-pentynyl, and the like.

The term "alkoxy" refers to an alkyl-O- group, wherein alkyl is as defined above. Examples of "C1-10 alkoxy" include methoxy, ethoxy, propoxy, isopropoxy, butoxy, isobutoxy, sec-butoxy, tertbutoxy, pentyloxy, hexyloxy, and the like. When halogenated, alkoxy may be substituted with 1-7, preferably 1-5 halogen atoms, specific examples include difluoromethoxy, trifluoromethoxy, 2,2,2-trifluoroethoxy, 4,4,4-trifluorobutoxy, and the like.

The term "cycloalkyl" refers to a cyclic saturated hydrocarbon group. Examples of C3-10 cycloalkyl include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, and adamantyl.

The term "aryl" refers to aromatic monocarbocyclic and polycarbocyclic ring systems, wherein individual carbocyclic rings in the polycyclic system are fused or linked to each other by single bonds. Suitable aryl groups include phenyl, naphthyl, 2,3-dihydro-1H-indenyl, and biphenyl. 6- to 14-membered aryl refers to a 6-carbon monocyclic, 10-carbon bicyclic, 12-carbon biphenyl, or 14-carbon tricyclic aromatic ring system.

The term "heteroaryl" refers to an aromatic 5- to 8-membered monocyclic, 8- to 12-membered bicyclic, or 11- to 14-membered tricyclic ring system, which has 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 O, N, or S heteroatoms for monocyclic, bicyclic, or tricyclic, respectively), wherein 0, 1, 2, 3, or 4 atoms of each ring are substituted with substituents. Examples of heteroaryl include pyridyl, furyl, imidazolyl, benzimidazolyl, pyrimidinyl, thienyl, quinolinyl, indolyl, thiazolyl, and the like.

The term "heterocyclyl" refers to a non-aromatic monocyclic, bicyclic, or tricyclic ring system. Thus, 5- to 14-membered heterocyclyl refers to a 5- to 8-membered monocyclic, 8- to 12-membered bicyclic, or 11- to 14-membered tricyclic ring system, which has 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 O, N, or S heteroatoms for monocyclic, bicyclic, or tricyclic, respectively) or other suitable heteroatoms (such as P or Si), wherein 0, 1, 2, or 3 atoms of each ring are substituted with substituents. Examples of 5- to 14-membered heterocyclyl include piperazinyl, pyrrolidinyl, dioxanyl, morpholinyl, tetrahydrofuranyl, tetrahydropyrrolidinyl, and the like.

The term "therapeutically effective amount" refers to an amount of a lipid-oligonucleotide conjugate of the present invention or a composition thereof that is effective to produce certain desired therapeutic effects in at least a subpopulation of cells in an animal at a reasonable benefit/risk ratio applicable to any medical treatment.

The term "pharmaceutically acceptable" refers to compounds, materials, compositions, and/or dosage forms that are, within the scope of sound medical judgment, suitable for contact with tissues of humans and animals without excessive toxicity, irritation, allergic reactions, or other problems or complications and commensurate with a reasonable benefit/risk ratio.

The term "pharmaceutically acceptable carrier" refers to a pharmaceutically acceptable material, composition, or vehicle that participates in carrying or transporting a lipid-oligonucleotide conjugate from one organ or part of the body to another organ or part of the body, such as a liquid or solid filler, diluent, excipient, manufacturing aid, or solvent encapsulating material. Each carrier must be "acceptable" in the sense of being compatible with other ingredients of the composition and innocuous to the patient.

The term "treatment" encompasses prevention, therapy, and cure. The patient receiving such treatment is typically any animal in need, including primates (particularly humans) and other mammals such as horses, cattle, swine, sheep, poultry, and companion animals.

### Lipid-Oligonucleotide Conjugates

A first aspect of the present invention provides a lipid-oligonucleotide conjugate.

In some embodiments, the present invention provides a lipid-oligonucleotide conjugate comprising a structure of Formula Ia: 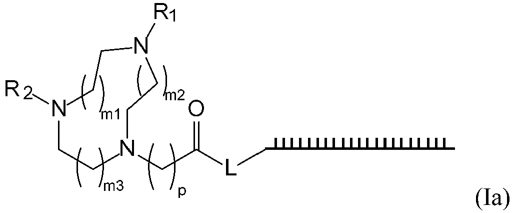 wherein,
at least one of R1 and R2 is selected from Ra, -C(O)Ra, -C(O)NHRa, and C(O)ORa, wherein Ra is optionally substituted C5-30 alkyl or alkenyl, and
the other of R1 and R2 is selected from H, Rb, -C(O)Rb, -C(O)NHRb, and C(O)ORb, wherein Rb is selected from optionally substituted C1-4 alkyl or alkenyl, optionally substituted C5-30 alkyl or alkenyl, optionally substituted C2-10 alkynyl, optionally substituted C1-10 alkoxy, optionally substituted C3-10 cycloalkyl, optionally substituted 6- to 14-membered aryl, optionally substituted 5- to 18-membered heteroaryl, optionally substituted 5- to 14-membered heterocyclyl;
said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5-to 18-membered heteroaryl, 5- to 14-membered heterocyclyl, halogen, hydroxyl, amino, guanidino, carboxyl, cyano, nitro, C1-3 alkylthio, and mercapto;
m1, m2, and m3 are each independently an integer of 1 or 2;
p is 0, 1, or 2; 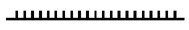
represents an oligonucleotide;
L represents a linker.

In some embodiments, one of R1 and R2 is -C(O)Ra, wherein Ra is optionally substituted C5-30 alkyl or alkenyl, and the other of R1 and R2 is H or -C(O)C1-4 alkyl.

In some embodiments, R1 is -C(O)Ra, wherein Ra is optionally substituted C5-30 alkyl or alkenyl, and R2 is H or -C(O)C1-4 alkyl.

In some embodiments, R2 is -C(O)Ra, wherein Ra is optionally substituted C5-30 alkyl or alkenyl, and R1 is H or -C(O)C1-4 alkyl.

In preferred embodiments, Ra is C5-30 alkyl or alkenyl. In preferred embodiments, Ra is C12-24 alkyl or alkenyl. In preferred embodiments, Ra is C14-22 alkyl or alkenyl. In preferred embodiments, Ra is C14, C15, C16, C18, C20, or C22 alkyl, monounsaturated alkenyl, or polyunsaturated alkenyl.

For example, Ra is selected from C5 alkyl, C6 alkyl, C7 alkyl, C8 alkyl, C9 alkyl, C10 alkyl, C11 alkyl, C12 alkyl, C13 alkyl, C14 alkyl, C15 alkyl, C16 alkyl, C17 alkyl, C18 alkyl, C19 alkyl, C20 alkyl, C21 alkyl, C22 alkyl, C23 alkyl, C24 alkyl, C25 alkyl, C26 alkyl, C27 alkyl, C28 alkyl, C29 alkyl, C30 alkyl, C5 monounsaturated alkenyl, C6 monounsaturated alkenyl, C7 monounsaturated alkenyl, C8 monounsaturated alkenyl, C9 monounsaturated alkenyl, C10 monounsaturated alkenyl, C11 monounsaturated alkenyl, C12 monounsaturated alkenyl, C13 monounsaturated alkenyl, C14 monounsaturated alkenyl, C15 monounsaturated alkenyl, C16 monounsaturated alkenyl, C17 monounsaturated alkenyl, C18 monounsaturated alkenyl, C19 monounsaturated alkenyl, C20 monounsaturated alkenyl, C21 monounsaturated alkenyl, C22 monounsaturated alkenyl, C23 monounsaturated alkenyl, C24 monounsaturated alkenyl, C25 monounsaturated alkenyl, C26 monounsaturated alkenyl, C27 monounsaturated alkenyl, C28 monounsaturated alkenyl, C29 monounsaturated alkenyl, C30 monounsaturated alkenyl, C5 polyunsaturated alkenyl, C6 polyunsaturated alkenyl, C7 polyunsaturated alkenyl, C8 polyunsaturated alkenyl, C9 polyunsaturated alkenyl, C10 polyunsaturated alkenyl, C11 polyunsaturated alkenyl, C12 polyunsaturated alkenyl, C13 polyunsaturated alkenyl, C14 polyunsaturated alkenyl, C15 polyunsaturated alkenyl, C16 polyunsaturated alkenyl, C17 polyunsaturated alkenyl, C18 polyunsaturated alkenyl, C19 polyunsaturated alkenyl, C20 polyunsaturated alkenyl, C21 polyunsaturated alkenyl, C22 polyunsaturated alkenyl, C23 polyunsaturated alkenyl, C24 polyunsaturated alkenyl, C25 polyunsaturated alkenyl, C26 polyunsaturated alkenyl, C27 polyunsaturated alkenyl, C28 polyunsaturated alkenyl, C29 polyunsaturated alkenyl, and C30 polyunsaturated alkenyl.

In preferred embodiments, both of R1 and R2 are and independently are -C(O)Ra, wherein Ra is optionally substituted C5-30 alkyl or alkenyl. In preferred embodiments, both of R1 and R2 are -C(O)Ra, and Ra is optionally substituted C5-30 alkyl or alkenyl. In preferred embodiments, R1 and R2 are the same and Ra is C5-30 alkyl or alkenyl. In preferred embodiments, R1 and R2 are the same and Ra is C12-24 alkyl or alkenyl. In preferred embodiments, R1 and R2 are the same and Ra is C14-22 alkyl or alkenyl. In preferred embodiments, R1 and R2 are the same and Ra is C14, C15, C16, C18, C20, or C22 alkyl, monounsaturated alkenyl, or polyunsaturated alkenyl.

In preferred embodiments, R1 and R2 are the same and Ra is selected from C5 alkyl, C6 alkyl, C7 alkyl, C8 alkyl, C9 alkyl, C10 alkyl, C11 alkyl, C12 alkyl, C13 alkyl, C14 alkyl, C15 alkyl, C16 alkyl, C17 alkyl, C18 alkyl, C19 alkyl, C20 alkyl, C21 alkyl, C22 alkyl, C23 alkyl, C24 alkyl, C25 alkyl, C26 alkyl, C27 alkyl, C28 alkyl, C29 alkyl, C30 alkyl, C5 monounsaturated alkenyl, C6 monounsaturated alkenyl, C7 monounsaturated alkenyl, C8 monounsaturated alkenyl, C9 monounsaturated alkenyl, C10 monounsaturated alkenyl, C11 monounsaturated alkenyl, C12 monounsaturated alkenyl, C13 monounsaturated alkenyl, C14 monounsaturated alkenyl, C15 monounsaturated alkenyl, C16 monounsaturated alkenyl, C17 monounsaturated alkenyl, C18 monounsaturated alkenyl, C19 monounsaturated alkenyl, C20 monounsaturated alkenyl, C21 monounsaturated alkenyl, C22 monounsaturated alkenyl, C23 monounsaturated alkenyl, C24 monounsaturated alkenyl, C25 monounsaturated alkenyl, C26 monounsaturated alkenyl, C27 monounsaturated alkenyl, C28 monounsaturated alkenyl, C29 monounsaturated alkenyl, C30 monounsaturated alkenyl, C5 polyunsaturated alkenyl, C6 polyunsaturated alkenyl, C7 polyunsaturated alkenyl, C8 polyunsaturated alkenyl, C9 polyunsaturated alkenyl, C10 polyunsaturated alkenyl, C11 polyunsaturated alkenyl, C12 polyunsaturated alkenyl, C13 polyunsaturated alkenyl, C14 polyunsaturated alkenyl, C15 polyunsaturated alkenyl, C16 polyunsaturated alkenyl, C17 polyunsaturated alkenyl, C18 polyunsaturated alkenyl, C19 polyunsaturated alkenyl, C20 polyunsaturated alkenyl, C21 polyunsaturated alkenyl, C22 polyunsaturated alkenyl, C23 polyunsaturated alkenyl, C24 polyunsaturated alkenyl, C25 polyunsaturated alkenyl, C26 polyunsaturated alkenyl, C27 polyunsaturated alkenyl, C28 polyunsaturated alkenyl, C29 polyunsaturated alkenyl, and C30 polyunsaturated alkenyl.

In some embodiments, m1, m2, and m3 are all 1. In some embodiments, two of m1, m2, and m3 are 1 and the other is 2. In some embodiments, m1 and m2 are 1, and m3 is 2. In some embodiments, m1 and m3 are 1, and m2 is 2. In some embodiments, m2 and m3 are 1, and m1 is 2.

In some embodiments, p is 2. In some embodiments, p is 0. In preferred embodiments, p is 1.

In some embodiments, the oligonucleotide is an antisense oligonucleotide or the sense strand or antisense strand of an RNAi agent. In some embodiments, the sense strand or antisense strand of said RNAi agent pairs with its antisense strand or sense strand to form a duplex. In preferred embodiments, said RNAi agent is siRNA.

In preferred embodiments, the oligonucleotide is an antisense oligonucleotide (ASO). In preferred embodiments, the oligonucleotide is the sense strand or antisense strand of siRNA. In preferred embodiments, the oligonucleotide is the sense strand of siRNA. In preferred embodiments, the sense strand or antisense strand of said siRNA pairs with its antisense strand or sense strand to form a duplex.

In some embodiments, the oligonucleotide may be conjugated via the 5' and/or 3' end. In certain embodiments, the oligonucleotide is conjugated via the 5' end, e.g., the 5' end of an ASO or the sense strand of siRNA. In certain embodiments, the oligonucleotide is conjugated via the 3' end, e.g., the 3' end of an ASO or the sense strand of siRNA. In alternative embodiments, the oligonucleotide is conjugated via 1, 2, 3, or 4 nucleotides before the 5' or 3' terminal nucleotide. In some embodiments, the oligonucleotide is conjugated via the 2' position of the sugar of the 3'-terminal nucleotide. In other embodiments, the oligonucleotide is conjugated via the 2' position of the sugar of the 5'-terminal nucleotide.

In preferred embodiments, the 5' and/or 3' end of said oligonucleotide is linked to L via a phosphate group, phosphorothioate group, or phosphonate group. In preferred embodiments, the 5' and/or 3' end of said ASO or siRNA is linked to L via a phosphorothioate group.

In preferred embodiments, the antisense strand of said siRNA has a 5'-VP modification.

"Linker" or L refers to an atom or group of atoms that covalently connects the lipid moiety to the oligonucleotide moiety. The linker may have a length of about 1 to about 30 atoms, about 2 to about 28 atoms, about 3 to about 26 atoms, about 4 to about 24 atoms, about 6 to about 20 atoms, about 7 to about 20 atoms, about 8 to about 20 atoms, about 8 to about 18 atoms, and about 12 to about 18 atoms. In some embodiments, the linker may comprise a bifunctional linking moiety, which generally includes an alkyl moiety having two functional groups. One functional group is selected for attachment to the compound of interest (e.g., the sense or antisense strand of an RNAi agent), while the other functional group is selected for attachment to essentially any selected group, such as the lipid moiety described herein. In certain embodiments, the linker includes an oligomer of chain structures or repeating units, such as ethylene glycol or amino acid units. Examples of functional groups commonly used in bifunctional linking moieties include, but are not limited to, electrophiles for reaction with nucleophilic groups and nucleophiles for reaction with electrophilic groups. In some embodiments, the bifunctional linking moiety includes amino, hydroxyl, carboxylic acid, thiol, unsaturated bonds (e.g., double or triple bonds), and the like.

Linkers useful for attaching lipids to oligonucleotides include, but are not limited to, pyrrolidine, 8-amino-3,6-dioxaoctanoic acid, N-succinimidyl-4-(N-maleimidomethyl)cyclohexane-1-carboxylate, 6-aminohexanoic acid, substituted C1-C20 alkyl, substituted or unsubstituted C2-C20 alkenyl, or substituted or unsubstituted C2-C20 alkynyl. Preferred substituents for such linkers include, but are not limited to, hydroxy, amino, alkoxy, carboxy, benzyl, phenyl, nitro, thiol, thioalkoxy, halogen, alkyl, aryl, alkenyl, and alkynyl.

In certain embodiments, the linker is cleavable. A cleavable linker is one that is sufficiently stable outside the cell but is cleaved upon entry into the target cell to release the two moieties joined together by the linker. In some embodiments, the cleavable linker is cleaved at least 10-fold, 20-fold, 30-fold, 40-fold, 50-fold, 60-fold, 70-fold, 80-fold, 90-fold or more, or at least 100-fold faster in the target cell or under a first reference condition (which may, for example, be selected to mimic or represent intracellular conditions) than in the blood of the subject.

Cleavable linkers are susceptible to cleavage agents, such as pH, redox potential, or the presence of degradative molecules. Generally, cleavage agents are more prevalent or found at higher levels or activity inside cells than in serum or blood. Examples of such cleavage agents include: redox agents, selected for specific substrates or lacking substrate specificity, including, for example, oxidases or reductases present in cells; esterases; endosomes or agents that can create an acidic environment, such as those resulting in a pH of 5 or lower; enzymes that can hydrolyze or degrade acid-cleavable linkers acting as general acids, peptidases (which can be substrate-specific), and phosphatases.

A cleavable linker can comprise a pH-sensitive moiety. Human serum has a pH of 7.4, while the average intracellular pH is slightly lower, ranging from about 7.1-7.3. Endosomes have a more acidic pH, in the range of 5.5-6.0, and lysosomes have an even more acidic pH (around 5.0). Some linkers will have a cleavable group that is cleaved at a preferred pH, thereby releasing the RNA molecule from the ligand inside the cell, or into a desired cellular organelle. The linker can include a cleavable group that is cleaved by a specific enzyme. The type of cleavable group incorporated into the linker can depend on the cell to be targeted.

Other types of linkers suitable for attaching lipids to oligonucleotides are known in the art, such as those described in CN116133691A, WO2023245061A2, or WO2017053995A1, all of which are incorporated herein by reference in their entireties.

In some embodiments, L is -NH-(CH2)q-O-, -NH-(CH2CH2O)q-, 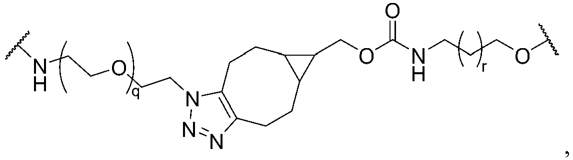 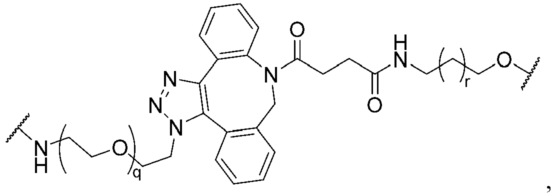 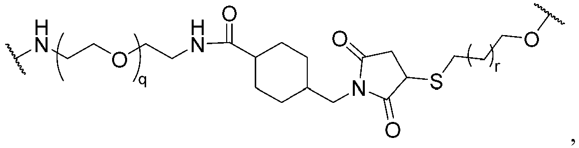 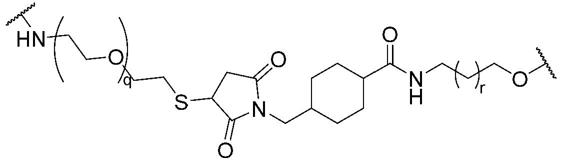 or 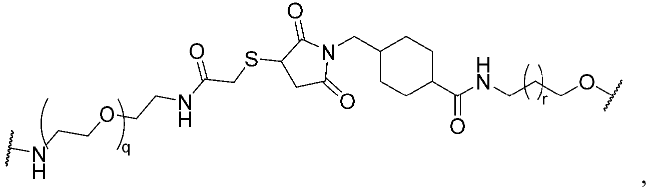 wherein q and r are independently integers from 1 to 20, and the right side of the formula is conjugated to the oligonucleotide. For example, q and r may independently be integers from 1 to 15, 1 to 10, 1 to 8, 1 to 6, 2 to 10, 3 to 10, 4 to 10, 5 to 10, 6 to 10, 10 to 15, or 10 to 20. For example, q and r may independently be 1, 2, 3, 4, 5, 6, 7, 8, 9, 10, 11, 12, 13, 14, 15, 16, 17, 18, 19, or 20.

In some embodiments, L is -NH-(CH2)3-O-, -NH-(CH2)4-O-, -NH-(CH2)5-O-, -NH-(CH2)6-O-, -NH-(CH2)7-O-, -NH-(CH2)8-O-, -NH-(CH2)9-O-, -NH-(CH2)10-O-, -NH-(CH2CH2O)-, -NH-(CH2CH2O)2-, -NH-(CH2CH2O)3-, -NH-(CH2CH2O)4-, -NH-(CH2CH2O)5-, or -NH-(CH2CH2O)6-.

In some embodiments, L comprises L1-L2, wherein L1 is 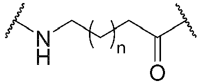 or 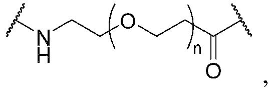 wherein the right side of the formula is connected to L2; L2 is 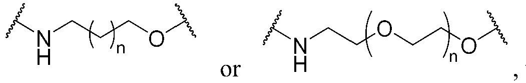 wherein the left side of the formula is connected to L1; the wavy line represents the connection position; and wherein each n is independently an integer from 1 to 10, preferably an integer from 2 to 8, more preferably an integer from 4 to 6. For example, each n may independently be 1, 2, 3, 4, 5, 6, 7, 8, 9, or 10.

In some embodiments, L comprises or is: 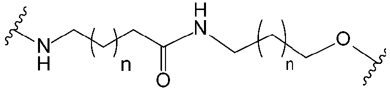 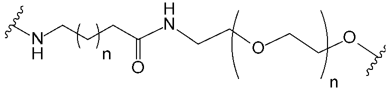 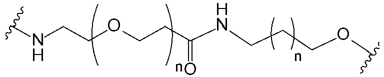 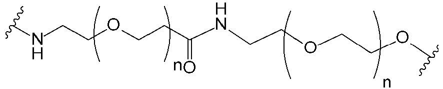 wherein the wavy lines represent connection positions, and the right-side wavy line is connected to the oligonucleotide, preferably to a phosphate, phosphorothioate, or phosphonate group at the 5' and/or 3' end of the oligonucleotide, and each n in each formula is independently an integer from 1 to 10, preferably from 2 to 8, more preferably from 4 to 6.

In some embodiments, the present invention provides a lipid-oligonucleotide conjugate represented by Formula Ib, 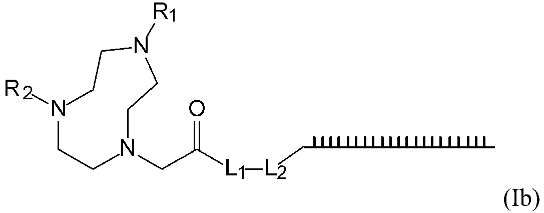 wherein,
at least one of R1 and R2 is selected from Ra, -C(O)Ra, -C(O)NHRa, and C(O)ORa, wherein Ra is optionally substituted C5-30 alkyl or alkenyl, and
the other of R1 and R2 is selected from H, Rb, -C(O)Rb, -C(O)NHRb, and C(O)ORb, wherein Rb is selected from optionally substituted C1-4 alkyl or alkenyl, optionally substituted C5-30 alkyl or alkenyl, optionally substituted C2-10 alkynyl, optionally substituted C1-10 alkoxy, optionally substituted C3-10 cycloalkyl, optionally substituted 6- to 14-membered aryl, optionally substituted 5- to 18-membered heteroaryl, optionally substituted 5- to 14-membered heterocyclyl;
said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5-to 18-membered heteroaryl, 5- to 14-membered heterocyclyl, halogen, hydroxyl, amino, guanidino, carboxyl, cyano, nitro, C1-3 alkylthio, and mercapto;
L1 is 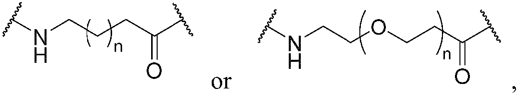 wherein the right side of the formula is connected to L2; L2 is 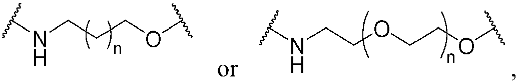 wherein the left side of the formula is connected to L1; the wavy line represents the connection position; and wherein each n is independently an integer from 1 to 10; 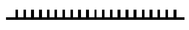
represents an oligonucleotide.

In some embodiments, the present invention provides a lipid-oligonucleotide conjugate represented by Formula Ib, 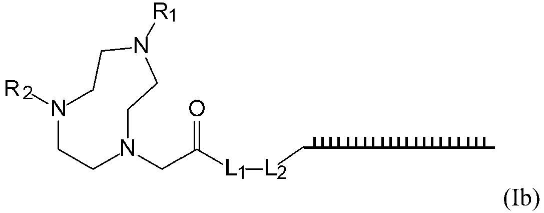 wherein
both of R1 and R2 are and independently are -C(O)Ra, wherein Ra is optionally substituted C5-30 alkyl or alkenyl;
L1 is 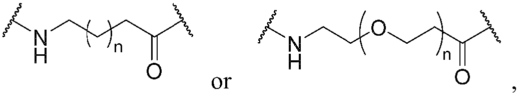 wherein the right side of the formula is connected to L2; L2 is 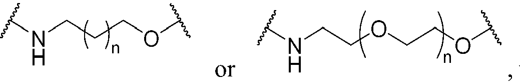 wherein the left side of the formula is connected to L1; the wavy line represents the connection position; and wherein each n is independently an integer from 1 to 10; 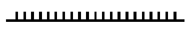
represents an oligonucleotide.

In some embodiments, the present invention provides a lipid-oligonucleotide conjugate represented by Formula Ib, 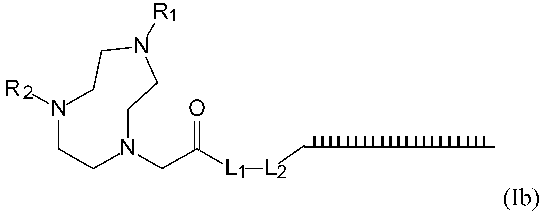 wherein,
both of R1 and R2 are and independently are -C(O)Ra, and Ra is C5-30 alkyl or alkenyl, preferably C12-24 alkyl or alkenyl, more preferably C14-22 alkyl or alkenyl, still more preferably C14, C15, C16, C18, C20, or C22 alkyl, monounsaturated alkenyl, or polyunsaturated alkenyl;
L1 is 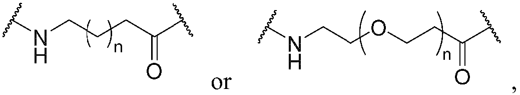 wherein the right side of the formula is connected to L2; L2 is 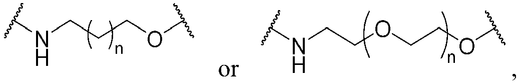 wherein the left side of the formula is connected to L1; the wavy line represents the connection position; and wherein each n is independently an integer from 1 to 10, preferably from 2 to 8, more preferably from 4 to 6; 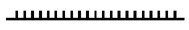
represents an oligonucleotide, preferably the oligonucleotide is an antisense oligonucleotide or the sense strand or antisense strand of an RNAi agent; preferably the 5' and/or 3' end of said oligonucleotide is linked to L2 via a phosphate group, phosphorothioate group, or phosphonate group; preferably the sense strand or antisense strand of said RNAi agent pairs with its antisense strand or sense strand to form a duplex; preferably said RNAi agent is siRNA; preferably said oligonucleotide is the sense strand of siRNA; preferably the antisense strand of said siRNA has a 5'-VP modification.

In preferred embodiments, the present invention provides a lipid-oligonucleotide conjugate represented by Formula Ic, 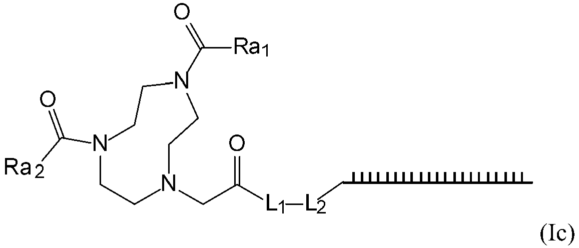 wherein,
one of Ra1 and Ra2 is optionally substituted C5-30 alkyl or alkenyl, preferably optionally substituted C12-24 alkyl or alkenyl; preferably optionally substituted C14-22 alkyl or alkenyl; more preferably optionally substituted C14, C15, C16, C18, C20, or C22 alkyl, monounsaturated alkenyl, or polyunsaturated alkenyl;
the other of Ra1 and Ra2 is optionally substituted C1-4 alkyl or alkenyl, optionally substituted C5-30 alkyl or alkenyl, optionally substituted C2-10 alkynyl, optionally substituted C1-10 alkoxy, optionally substituted C3-10 cycloalkyl, optionally substituted 6- to 14-membered aryl, optionally substituted 5- to 18-membered heteroaryl, or optionally substituted 5- to 14-membered heterocyclyl;
said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5-to 18-membered heteroaryl, 5- to 14-membered heterocyclyl, halogen, hydroxyl, amino, guanidino, carboxyl, cyano, nitro, C1-3 alkylthio, and mercapto;
L1 is 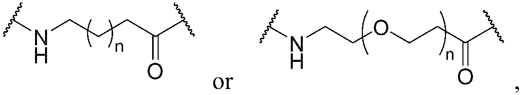 wherein the right side of the formula is connected to L2; L2 is 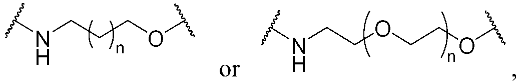 wherein the left side of the formula is connected to L1; the wavy line represents the connection position; and wherein each n is independently an integer from 1 to 10; 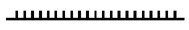
represents an oligonucleotide.

In preferred embodiments, the present invention provides a lipid-oligonucleotide conjugate represented by Formula Ic, 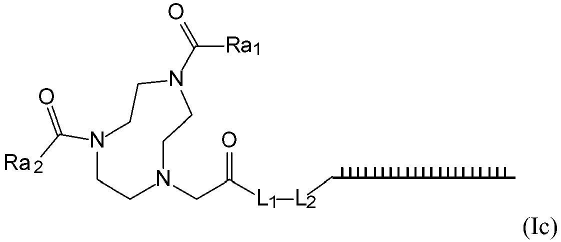 wherein,
one of Ra1 and Ra2 is C5-30 alkyl or alkenyl, preferably C12-24 alkyl or alkenyl, more preferably C14-22 alkyl or alkenyl, still more preferably C14, C15, C16, C18, C20, or C22 alkyl, monounsaturated alkenyl, or polyunsaturated alkenyl;
the other of Ra1 and Ra2 is C1-4 alkyl or alkenyl, C5-30 alkyl or alkenyl, C2-10 alkynyl, C1-10 alkoxy, C3-10 cycloalkyl, 6- to 14-membered aryl, 5- to 18-membered heteroaryl, or 5- to 14-membered heterocyclyl;
L1 is 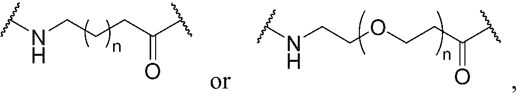 wherein the right side of the formula is connected to L2; L2 is 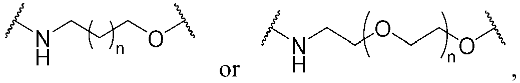 wherein the left side of the formula is connected to L1; the wavy line represents the connection position; and wherein each n is independently an integer from 1 to 10; 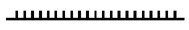
represents an oligonucleotide.

In preferred embodiments, in Formula Ic, one of Ra1 and Ra2 is selected from C1-4 alkyl or alkenyl, and the other is selected from C5-30 alkyl or alkenyl. In these embodiments, one of Ra1 and Ra2 (e.g., Ra1) is selected from methyl, ethyl, propyl, isopropyl, butyl, isobutyl, sec-butyl, tert-butyl, vinyl, propenyl, 1-butenyl, isobutenyl, cis-2-butenyl, or trans-2-butenyl, and the other (e.g., Ra2) is selected from C5 alkyl, C6 alkyl, C7 alkyl, C8 alkyl, C9 alkyl, C10 alkyl, C11 alkyl, C12 alkyl, C13 alkyl, C14 alkyl, C15 alkyl, C16 alkyl, C17 alkyl, C18 alkyl, C19 alkyl, C20 alkyl, C21 alkyl, C22 alkyl, C23 alkyl, C24 alkyl, C25 alkyl, C26 alkyl, C27 alkyl, C28 alkyl, C29 alkyl, C30 alkyl, C5 monounsaturated alkenyl, C6 monounsaturated alkenyl, C7 monounsaturated alkenyl, C8 monounsaturated alkenyl, C9 monounsaturated alkenyl, C10 monounsaturated alkenyl, C11 monounsaturated alkenyl, C12 monounsaturated alkenyl, C13 monounsaturated alkenyl, C14 monounsaturated alkenyl, C15 monounsaturated alkenyl, C16 monounsaturated alkenyl, C17 monounsaturated alkenyl, C18 monounsaturated alkenyl, C19 monounsaturated alkenyl, C20 monounsaturated alkenyl, C21 monounsaturated alkenyl, C22 monounsaturated alkenyl, C23 monounsaturated alkenyl, C24 monounsaturated alkenyl, C25 monounsaturated alkenyl, C26 monounsaturated alkenyl, C27 monounsaturated alkenyl, C28 monounsaturated alkenyl, C29 monounsaturated alkenyl, C30 monounsaturated alkenyl, C5 polyunsaturated alkenyl, C6 polyunsaturated alkenyl, C7 polyunsaturated alkenyl, C8 polyunsaturated alkenyl, C9 polyunsaturated alkenyl, C10 polyunsaturated alkenyl, C11 polyunsaturated alkenyl, C12 polyunsaturated alkenyl, C13 polyunsaturated alkenyl, C14 polyunsaturated alkenyl, C15 polyunsaturated alkenyl, C16 polyunsaturated alkenyl, C17 polyunsaturated alkenyl, C18 polyunsaturated alkenyl, C19 polyunsaturated alkenyl, C20 polyunsaturated alkenyl, C21 polyunsaturated alkenyl, C22 polyunsaturated alkenyl, C23 polyunsaturated alkenyl, C24 polyunsaturated alkenyl, C25 polyunsaturated alkenyl, C26 polyunsaturated alkenyl, C27 polyunsaturated alkenyl, C28 polyunsaturated alkenyl, C29 polyunsaturated alkenyl, and C30 polyunsaturated alkenyl.

In preferred embodiments, in Formula Ic, Ra1 and Ra2 are independently selected from C5 alkyl, C6 alkyl, C7 alkyl, C8 alkyl, C9 alkyl, C10 alkyl, C11 alkyl, C12 alkyl, C13 alkyl, C14 alkyl, C15 alkyl, C16 alkyl, C17 alkyl, C18 alkyl, C19 alkyl, C20 alkyl, C21 alkyl, C22 alkyl, C23 alkyl, C24 alkyl, C25 alkyl, C26 alkyl, C27 alkyl, C28 alkyl, C29 alkyl, C30 alkyl, C5 monounsaturated alkenyl, C6 monounsaturated alkenyl, C7 monounsaturated alkenyl, C8 monounsaturated alkenyl, C9 monounsaturated alkenyl, C10 monounsaturated alkenyl, C11 monounsaturated alkenyl, C12 monounsaturated alkenyl, C13 monounsaturated alkenyl, C14 monounsaturated alkenyl, C15 monounsaturated alkenyl, C16 monounsaturated alkenyl, C17 monounsaturated alkenyl, C18 monounsaturated alkenyl, C19 monounsaturated alkenyl, C20 monounsaturated alkenyl, C21 monounsaturated alkenyl, C22 monounsaturated alkenyl, C23 monounsaturated alkenyl, C24 monounsaturated alkenyl, C25 monounsaturated alkenyl, C26 monounsaturated alkenyl, C27 monounsaturated alkenyl, C28 monounsaturated alkenyl, C29 monounsaturated alkenyl, C30 monounsaturated alkenyl, C5 polyunsaturated alkenyl, C6 polyunsaturated alkenyl, C7 polyunsaturated alkenyl, C8 polyunsaturated alkenyl, C9 polyunsaturated alkenyl, C10 polyunsaturated alkenyl, C11 polyunsaturated alkenyl, C12 polyunsaturated alkenyl, C13 polyunsaturated alkenyl, C14 polyunsaturated alkenyl, C15 polyunsaturated alkenyl, C16 polyunsaturated alkenyl, C17 polyunsaturated alkenyl, C18 polyunsaturated alkenyl, C19 polyunsaturated alkenyl, C20 polyunsaturated alkenyl, C21 polyunsaturated alkenyl, C22 polyunsaturated alkenyl, C23 polyunsaturated alkenyl, C24 polyunsaturated alkenyl, C25 polyunsaturated alkenyl, C26 polyunsaturated alkenyl, C27 polyunsaturated alkenyl, C28 polyunsaturated alkenyl, C29 polyunsaturated alkenyl, and C30 polyunsaturated alkenyl.

In preferred embodiments, in Formula Ic, Ra1 and Ra2 are the same.

In preferred embodiments, in Formula Ic, the oligonucleotide is an antisense oligonucleotide or the sense strand or antisense strand of an RNAi agent; preferably the 5' and/or 3' end of said oligonucleotide is linked to L2 via a phosphate group, phosphorothioate group, or phosphonate group; preferably the sense strand or antisense strand of said RNAi agent pairs with its antisense strand or sense strand to form a duplex; preferably said RNAi agent is siRNA; preferably said oligonucleotide is the sense strand of siRNA; preferably the antisense strand of said siRNA has a 5'-VP modification.

In some embodiments, in Formula Ic, L1-L2 together constitute: 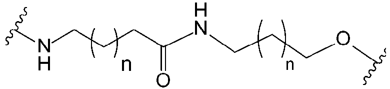 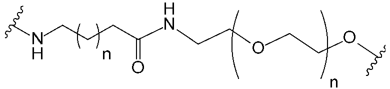 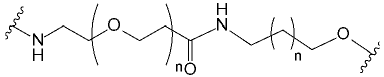 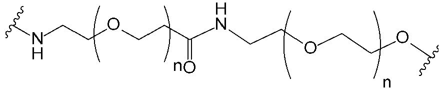 wherein the wavy lines represent connection positions, and the right-side wavy line is connected to the oligonucleotide, preferably to a phosphate, phosphorothioate, or phosphonate group at the 5' and/or 3' end of the oligonucleotide, and each n in each formula is independently an integer from 1 to 10, preferably from 2 to 8, more preferably from 4 to 6.

In some specific embodiments, the present invention provides lipid-oligonucleotide conjugates of the following structural formulae: 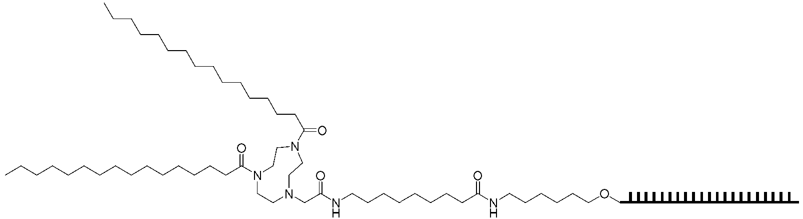 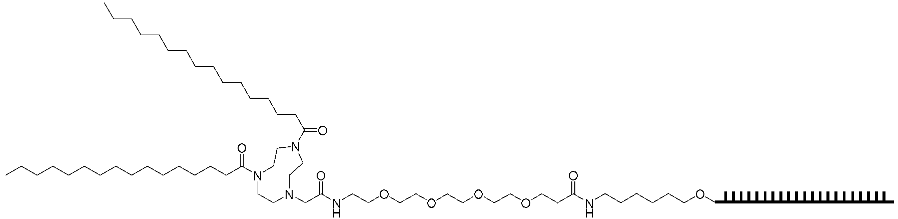 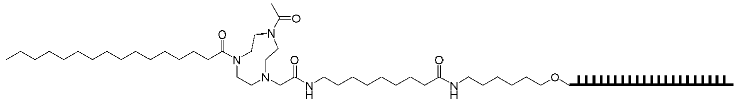 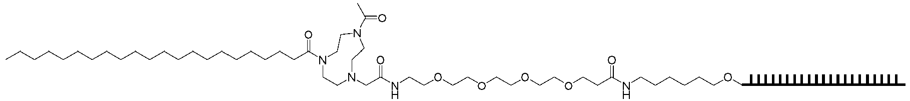 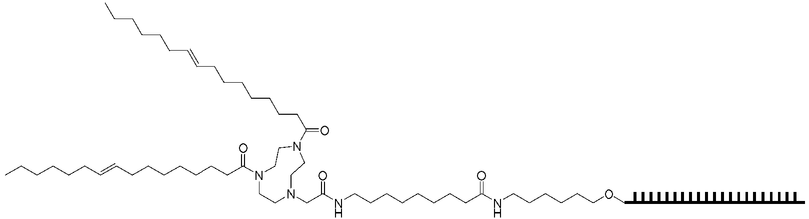 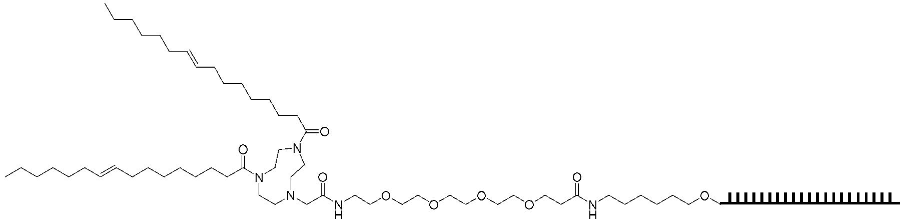 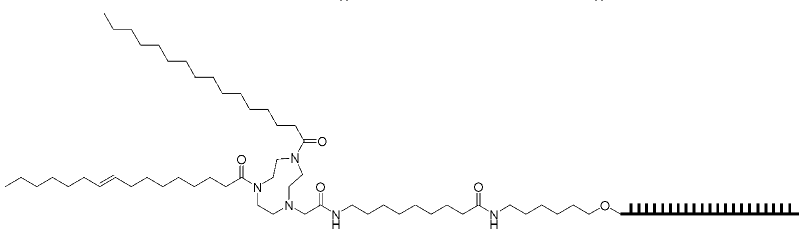 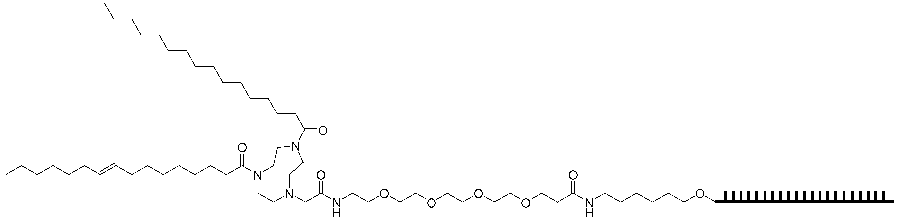 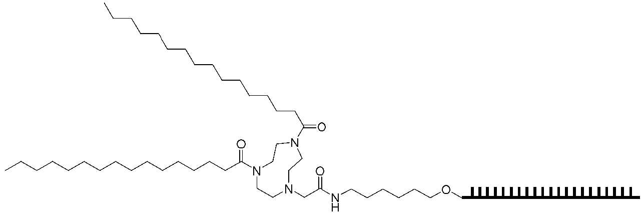 wherein 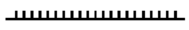 represents an oligonucleotide.

In preferred embodiments, said oligonucleotide is an antisense oligonucleotide or the sense strand or antisense strand of an RNAi agent; preferably the 5' and/or 3' end of said oligonucleotide is conjugated via a phosphate group, phosphorothioate group, or phosphonate group; preferably the sense strand or antisense strand of said RNAi agent pairs with its antisense strand or sense strand to form a duplex; preferably said RNAi agent is siRNA; preferably said oligonucleotide is the sense strand of siRNA; preferably the antisense strand of said siRNA has a 5'-VP modification.

In preferred embodiments, said lipid-oligonucleotide conjugate is selected from any one of the following structures: 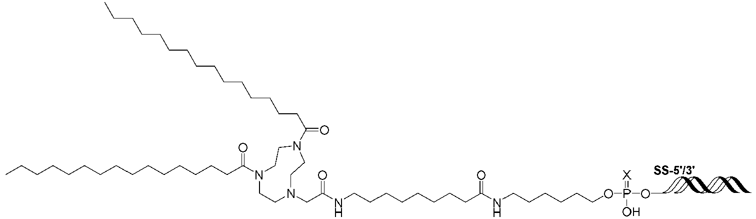 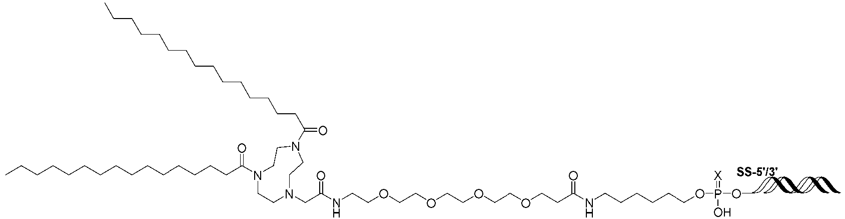 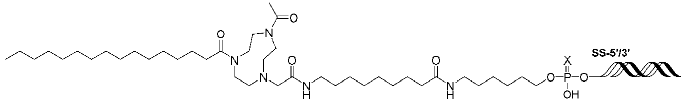 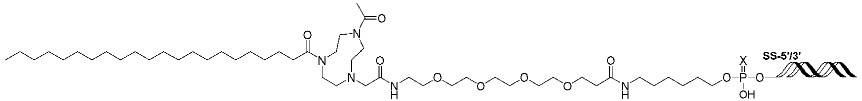 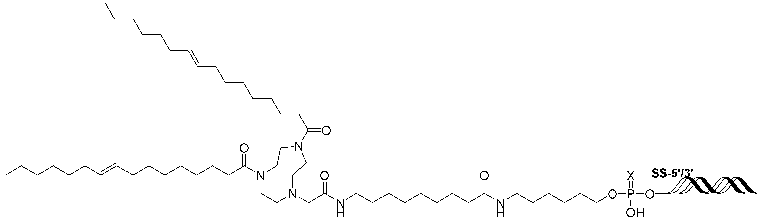 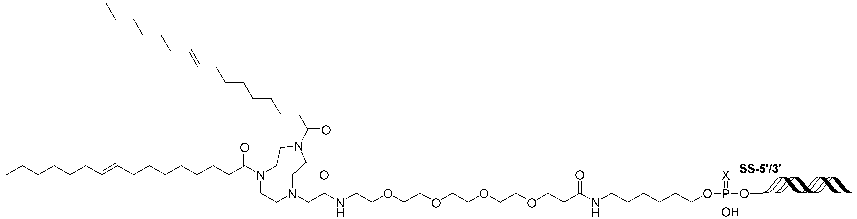 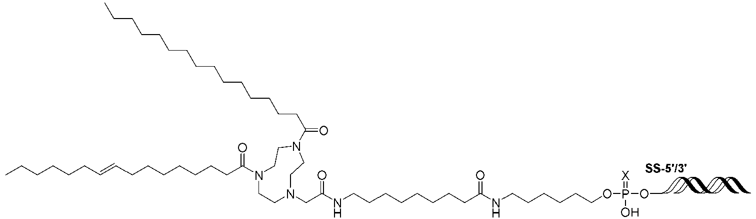 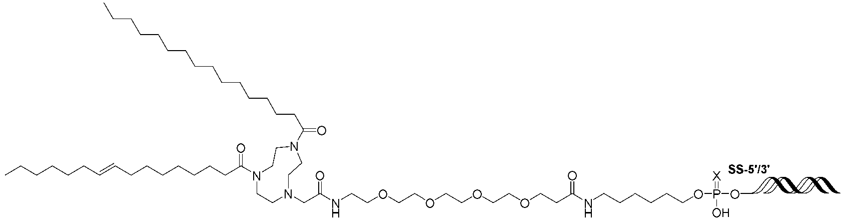 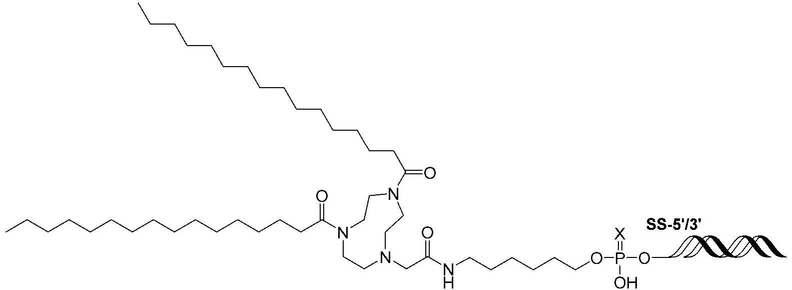 wherein, 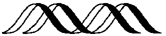 represents siRNA, X is S or O, and SS-5'/3' represents the 5' or 3' end of the sense strand.

In some embodiments, the oligonucleotide is an antisense oligonucleotide. In some embodiments, the provided antisense oligonucleotide comprises modified nucleotides, e.g., modified sugar moieties and/or modified nucleobase moieties and/or modified internucleotide linkages. In preferred embodiments, the provided antisense oligonucleotide comprises modified nucleotides, including modified sugar moieties, modified nucleobase moieties, and modified internucleotide linkages.

In some embodiments, the antisense oligonucleotide comprises an oligonucleotide consisting of 12-30, 14-22, 14-20, 14-18, 14-20, 15-17, 15-25, or 16-20 linked nucleosides, wherein the antisense oligonucleotide comprises at least one modification selected from the group consisting of a modified sugar moiety, a modified nucleobase moiety, and a modified internucleoside linkage.

In some embodiments, every nucleoside in the oligonucleotide comprised in the antisense oligonucleotide is modified, said modification comprising a modified sugar moiety and a modified internucleoside linkage.

In some embodiments, every nucleoside in the oligonucleotide comprised in the antisense oligonucleotide is modified, said modification comprising a modified sugar moiety, a modified nucleobase moiety, and a modified internucleoside linkage.

In some embodiments, the modified sugar moiety may be at least one bicyclic sugar moiety, at least one non-bicyclic modified sugar moiety, and/or at least one sugar surrogate.

In some embodiments, the antisense oligonucleotide comprises at least one bicyclic sugar moiety. In some embodiments, a substituent bridges two atoms of a furanosyl ring to form a second ring, thereby generating a bicyclic sugar moiety. In certain such embodiments, the bicyclic sugar moiety comprises a bridge between the 4' and 2' furanosyl ring atoms. Examples of such 4' to 2' bridging sugar substituents include, but are not limited to: 4'-CH₂-2', 4'-(CH₂)₂-2', 4'-(CH₂)₃-2', 4'-CH₂-O-2' ("LNA"), 4'-CH₂-S-2', 4'-(CH₂)₂-O-2' ("ENA"), 4'-CH(CH₃)-O-2' (referred to as "constrained ethyl" or "cEt"), 4'-CH₂-O-CH₂-2', 4'-CH₂-N(R)-2', 4'-CH(CH₂OCH₃)-O-2' ("constrained MOE" or "cMOE") and analogs thereof (see, e.g., Seth et al., U.S. 7,399,845; Bhat et al., U.S. 7,569,686; Swayze et al., U.S. 7,741,457; and Swayze et al., U.S. 8,022,193), 4'-C(CH₃)(CH₃)-O-2' and analogs thereof (see, e.g., Seth et al., U.S. 8,278,283), 4'-CH₂-N(OCH₃)-2' and analogs thereof (see, e.g., Prakash et al., U.S. 8,278,425), 4'-CH₂-O-N(CH₃)-2' (see, e.g., Allerson et al., U.S. 7,696,345 and Allerson et al., U.S. 8,124,745), 4'-CH₂-C(H)(CH₃)-2' (see, e.g., Zhou et al., J. Org. Chem., 2009, 74, 118-134), 4'-CH₂-C(=CH₂)-2' and analogs thereof (see, e.g., Seth et al., U.S. 8,278,426), 4'-C(RₐR_{b})-N(R)-O-2', 4'-C(RₐR_{b})-O-N(R)-2', 4'-CH₂-O-N(R)-2', and 4'-CH₂-N(R)-O-2', wherein each R, Rₐ, and R_{b} is independently H, a protecting group, or C₁-C₁₂ alkyl (see, e.g., Imanishi et al., U.S. 7,427,672). In certain embodiments, bicyclic sugar moieties and nucleosides incorporating such bicyclic sugar moieties are further defined by isomeric configuration. For example, LNA nucleosides (as described herein) may be in the α-L configuration or in the β-D configuration. In preferred embodiments, the bicyclic sugar moiety has a 4'-2' bridge, which is preferably cEt or LNA.

In some embodiments, the antisense oligonucleotide comprises at least one non-bicyclic modified sugar moiety, and such non-bridging substituents may be located at any position of the furanosyl ring, including, but not limited to, substituents at the 2'-position, the 4'-position, and/or the 5'-position. In certain embodiments, one or more non-bridging substituents of the non-bicyclic modified sugar moiety are branched. Examples of 2'-substituents suitable for non-bicyclic modified sugar moieties include, but are not limited to: 2'-F, 2'-OCH₃ ("OMe" or "O-methyl"), and 2'-O(CH₂)₂OCH₃ ("MOE" or "O-methoxyethyl"). In certain embodiments, the 2'-substituent is selected from: halo, allyl, amino, azido, SH, CN, OCN, CF₃, OCF₃, O-C₁-C₁₀ alkoxy, substituted O-C₁-C₁₀ alkoxy, O-C₁-C₁₀ alkyl, substituted O-C₁-C₁₀ alkyl, S-alkyl, N(Rₘ)-alkyl, O-alkenyl, S-alkenyl, N(Rₘ)-alkenyl, O-alkynyl, S-alkynyl, N(Rₘ)-alkynyl, O-alkylene-O-alkyl, alkynyl, alkaryl, aralkyl, O-alkaryl, O-aralkyl, O(CH₂)₂SCH₃, O(CH₂)₂ON(Rₘ)(Rₙ), or OCH₂C(=O)-N(Rₘ)(Rₙ), wherein each Rₘ and Rₙ is independently H, an amino protecting group, or substituted or unsubstituted C₁-C₁₀ alkyl, as well as the 2'-substituents set forth in Cook et al., U.S. 6,531,584; Cook et al., U.S. 5,859,221; and Cook et al., U.S. 6,005,087. Certain embodiments of these 2'-substituents may be further substituted with one or more substituents independently selected from: hydroxyl, amino, alkoxy, carboxyl, benzyl, phenyl, nitro (NO₂), thiol, thioalkoxy, thioalkyl, halogen, alkyl, aryl, alkenyl, and alkynyl. Examples of 4'-substituents suitable for non-bicyclic modified sugar moieties include, but are not limited to, alkoxy (e.g., methoxy), alkyl, and those groups set forth in Manoharan et al., WO 2015/106128. Examples of 5'-substituents suitable for non-bicyclic modified sugar moieties include, but are not limited to: 5'-methyl (R or S), 5'-vinyl, and 5'-methoxy. In certain embodiments, the non-bicyclic modified sugar moiety comprises more than one non-bridging sugar substituent, such as a 2'-F-5'-methyl sugar moiety and modified sugar moieties and modified nucleosides as set forth in Migawa et al., WO 2008/101157 and Rajeev et al., US 2013/0203836.

In certain embodiments, the 2'-substituted non-bicyclic modified nucleoside comprises a sugar moiety comprising a non-bridging 2'-substituent selected from the group consisting of: F, NH₂, N₃, OCF₃, OCH₃, O(CH₂)₃NH₂, CH₂CH=CH₂, OCH₂CH=CH₂, OCH₂CH₂OCH₃, O(CH₂)₂SCH₃, O(CH₂)₂ON(Rₘ)(Rₙ), O(CH₂)₂O(CH₂)₂N(CH₃)₂, and N-substituted acetamide (OCH₂C( = O)-N(Rₘ)(Rₙ)), wherein each Rₘ and Rₙ is independently H, an amino protecting group, or substituted or unsubstituted C₁-C₁₀ alkyl.

In certain embodiments, the 2'-substituted non-bicyclic modified nucleoside comprises a sugar moiety comprising a non-bridging 2'-substituent selected from the group consisting of: F, OCF₃, OCH₃, OC H₂CH₂OCH₃, O(CH₂)₂SCH₃, O(CH₂)₂ON(CH₃)₂, O(CH₂)₂O(CH₂)₂N(CH₃)₂, and OCH₂C(=O)-N(H)CH₃("NMA"). In certain embodiments, the 2'-substituted non-bicyclic modified nucleoside comprises a sugar moiety comprising a non-bridging 2'-substituent selected from the group consisting of: F (2'-F), OCH₃ (2'-OMe), and OCH₂CH₂OCH₃ (2'-MOE).

In certain embodiments, the modified furanosyl sugar moiety and the nucleoside incorporating such a modified furanosyl sugar moiety are further defined by isomeric configuration. For example, a 2'-deoxyfuranosyl sugar moiety may be in any of seven isomeric configurations other than the naturally occurring β-D-deoxyribosyl configuration. Such modified sugar moieties are set forth in, for example, WO 2019/157531, which is incorporated herein by reference. A 2'-modified sugar moiety has an additional stereocenter at the 2'-position relative to the 2'-deoxyfuranosyl sugar moiety; therefore, such sugar moieties have a total of sixteen possible isomeric configurations. Unless otherwise specified, the 2'-modified sugar moieties described herein are in the β-D-ribosyl isomeric configuration.

In some embodiments, the antisense oligonucleotide comprises at least one sugar surrogate. In certain such embodiments, the oxygen atom of the sugar moiety is replaced by, for example, a sulfur, carbon, or nitrogen atom. In certain such embodiments, such modified sugar moieties also comprise bridging and/or non-bridging substituents as set forth herein. For example, certain sugar surrogates comprise a 4'-sulfur atom and substituents at the 2'-position (see, e.g., Bhat et al., U.S. 7,875,733 and Bhat et al., U.S. 7,939,677) and/or the 5'-position.

In certain embodiments, the sugar surrogate comprises a ring having other than five atoms. For example, in certain embodiments, the sugar surrogate comprises a six-membered tetrahydropyran ("THP"). Such tetrahydropyrans may be further modified or substituted. Nucleosides comprising such modified tetrahydropyrans include, but are not limited to, hexitol nucleic acid ("HNA"), anitol nucleic acid ("ANA"), mannitol nucleic acid ("MNA") (see, e.g., Leumann, C. J. Bioorg. & Med. Chem. 2002, 10, 841-854), and fluoro HNA (F-HNA, also referred to as F-THP or 3'-fluoro tetrahydropyran).

In certain embodiments, the sugar surrogate comprises a ring having more than five atoms and more than one heteroatom. For example, nucleosides comprising a morpholino sugar moiety and their use in oligonucleotides have been reported. In certain embodiments, the sugar surrogate comprises an acyclic moiety. Examples of nucleosides and oligonucleotides comprising such acyclic sugar surrogates include, but are not limited to: peptide nucleic acid ("PNA"), acyclic butyl nucleic acid (see, e.g., Kumar et al., Org. Biomol. Chem., 2013, 11, 5853-5865), and the nucleosides and oligonucleotides set forth in Manoharan et al., WO 2011/133876. By introducing a modified nucleobase having a cationic lipid or its equivalent covalently linked thereto, PNA can be rendered highly permeable to mammalian cell membranes (see, e.g., WO 2019/022434 and WO 2009/113828). In preferred embodiments, the sugar surrogate is selected from POM, PNA, THP, and F-HNA.

In some embodiments, the antisense oligonucleotide comprises one or more nucleosides comprising an unmodified nucleobase. In certain embodiments, the antisense oligonucleotide comprises one or more nucleosides comprising a modified nucleobase. In certain embodiments, the antisense oligonucleotide comprises one or more nucleosides that do not comprise a nucleobase, referred to as abasic nucleosides.

In certain embodiments, the modified nucleobase is selected from: 5-substituted pyrimidines (e.g., 5-methylcytosine, m5C), 6-azapyrimidines, alkyl- or alkynyl-substituted pyrimidines, alkylsubstituted purines, and N-2, N-6, and O-6 substituted purines. In certain embodiments, the modified nucleobase is selected from: 2-aminopropyladenine, 5-hydroxymethylcytosine, xanthine, hypoxanthine, 2-aminoadenine, 6-N-methylguanine, 6-N-methyladenine, 2-propyladenine, 2-thiouracil, 2-thiothymine, and 2-thiocytosine, 5-propynyl (-C≡C-CH₃) uracil, 5-propynylcytosine, 6-azouracil, 6-azocytosine, 6-azothymine, 5-ribosyluracil (pseudouracil), 4-thiouracil; 8-halo-, 8-amino-, 8-thiol-, 8-thioalkyl-, 8-hydroxy-, 8-aza,- and other 8-substituted purines; 5-halo-, particularly 5-bromo-, 5-trifluoromethyl-, 5-halouracil, and 5-halocytosine; 7-methylguanine, 7-methyladenine, 2-F-adenine, 2-aminoadenine, 7-deazaguanine, 7-deazaadenine, 3-deazaguanine, 3-deazaadenine, 6-N-benzoyladenine, 2-N-isobutyrylguanine, 5-methylcytosine, 4-N-benzoylcytosine, 4-N-benzoyluracil, 5-methyl-4-N-benzoylcytosine, 5-methyl-4-N-benzoyluracil, universal bases, hydrophobic bases, promiscuous bases, size-expanded bases, and fluorinated bases. Other modified nucleobases include tricyclic pyrimidines, such as 1,3-diazaphenoxazine-2-one, 1,3-diazaphenothiazin-2-one, and 9-(2-aminoethoxy)-1,3-diazaphenoxazine-2-one (G-clamp). Modified nucleobases may also include those in which the purine or pyrimidine base is replaced by other heterocycles, such as 7-deaza-adenine, 7-deazaguanosine, 2-aminopyridine, and 2-pyridone. In preferred embodiments, the antisense oligonucleotide comprises one or more 5-methylcytosines (m5C).

In some embodiments, the modified nucleobase may be a nucleobase having a cationic lipid covalently linked thereto. By introducing a modified nucleobase having a cationic lipid or an equivalent thereof covalently linked thereto, the antisense oligonucleotide can be rendered highly permeable to mammalian cell membranes. Suitable cationic lipid-linked modifications covalently conjugated thereto may be those described in WO 2019022434A1, which is incorporated herein by reference in its entirety.

In some embodiments, the antisense oligonucleotide comprises at least one modified internucleoside linkage. In preferred embodiments, every internucleoside linkage of the antisense oligonucleotide is a modified internucleoside linkage. In certain embodiments, any internucleoside linkage may be used to link the nucleosides of the antisense oligonucleotide together. Two main classes of internucleoside linking groups are defined by the presence or absence of a phosphorus atom. Representative phosphorus-containing internucleoside linkages include, but are not limited to, phosphodiester, which contains a phosphodiester bond ("P(O₂)=O") (also referred to as an unmodified linkage or naturally occurring linkage); phosphotriester; methylphosphonate; phosphoramidate (PN); phosphorothioate ("P(O₂)=S", PS); and phosphorodithioate ("HS-P=S"). Phosphoramidate (PN) may have the structural formula - N(R)P(=X)(OH)O-, -OP(=X)(OH)N(R)-, -O-P(NR)(=X)O-, - N(SO2R)P(=X)(OH)O-, - OP(=X)(OH)N(SO₂R)-, or -O-P(NSO₂R)(=X)O- ("MsPA"), wherein X is O or S, R may be optionally substituted alkyl, aryl, heteroaryl, or heterocycloalkyl; or NR may be an optionally substituted cyclic guanidinyl moiety, an optionally substituted triazolyl group, or a Tmg group. Further PN structures can be found in WO 2023220744, WO 2019/032612, and WO 2021/030778, which are incorporated herein by reference in their entirety.

Representative phosphorus-free internucleoside linking groups include, but are not limited to, methylenemethylimino (-CH₂-N(CH₃)-O-CH₂-), thiodiester, thiocarbamate (-O-C(=O)(NH)-S-); siloxane (-O-SiH₂-O-); and N,N'-dimethylhydrazine (-CH₂-N(CH₃)-N(CH₃)-). Modified internucleoside linkages can be used to alter, and typically increase, the nuclease resistance of oligonucleotides compared to naturally occurring phosphodiester internucleoside linkages. In certain embodiments, internucleoside linkages having a chiral atom can be prepared as racemic mixtures or as separated enantiomers. Methods for preparing phosphorus-containing and phosphorus-free internucleoside linkages are well known to those skilled in the art.

Representative internucleoside linkages having a chiral center include, but are not limited to, alkylphosphonates and phosphorothioates. Antisense oligonucleotides comprising internucleoside linkages having a chiral center may be prepared as a population of antisense oligonucleotides comprising stereorandom internucleoside linkages, or as a population of antisense oligonucleotides comprising phosphorothioate internucleoside linkages in a specific stereochemical configuration. In certain embodiments, a population of antisense oligonucleotides comprises phosphorothioate internucleoside linkages, wherein all of said phosphorothioate internucleoside linkages are stereorandom. Such antisense oligonucleotides can be generated using synthetic methods that randomly select the stereochemical configuration of each phosphorothioate internucleoside linkage. Nevertheless, as is well understood by those skilled in the art, each individual phosphorothioate linkage of each individual oligonucleotide molecule has a defined stereochemical configuration. In certain embodiments, the population of antisense oligonucleotides is enriched for antisense oligonucleotides comprising one or more specific phosphorothioate internucleoside linkages in a specific, independently selected stereochemical configuration. In certain embodiments, at least 65% of the molecules in the population have the specific phosphorothioate internucleoside linkage in a specific configuration. In certain embodiments, at least 70% of the molecules in the population have the specific phosphorothioate internucleoside linkage in a specific configuration. In certain embodiments, at least 80% of the molecules in the population have the specific phosphorothioate internucleoside linkage in a specific configuration. In certain embodiments, at least 90% of the molecules in the population have the specific phosphorothioate internucleoside linkage in a specific configuration. In certain embodiments, at least 99% of the molecules in the population have the specific phosphorothioate internucleoside linkage in a specific configuration. Such chirally enriched populations of antisense oligonucleotides can be generated using synthetic methods known in the art, such as those set forth in Oka et al., JACS 125, 8307 (2003); Wan et al., Nuc. Acid. Res. 42, 13456 (2014); and WO 2017/015555. In certain embodiments, the population of antisense oligonucleotides is enriched for antisense oligonucleotides having at least one indicated phosphorothioate linkage in the (Sp) configuration. In certain embodiments, the population of antisense oligonucleotides is enriched for antisense oligonucleotides having at least one phosphorothioate linkage in the (Rp) configuration.

In some embodiments, the antisense oligonucleotide comprises a gapmer. Said gapmer is defined by two external regions or "wings" and a central or internal region or "gap." The three regions of the gapmer motif (5'-wing, gap, and 3'-wing) form a contiguous sequence of nucleosides, wherein at least some sugar moieties of the nucleosides of each wing are different from at least some sugar moieties of the nucleosides of the gap. Specifically, at least the sugar moiety of the nucleoside in each wing that is closest to the gap (the 3'-most nucleoside of the 5'-wing and the 5'-most nucleoside of the 3'-wing) is different from the sugar moiety of the adjacent gap nucleoside, thereby defining the boundary between the wing and the gap (i.e., the wing/gap junction). In certain embodiments, the sugar moieties within the gap are identical to each other. In certain embodiments, the gap comprises one or more nucleosides having a sugar moiety that is different from the sugar moiety of one or more other nucleosides of the gap. In certain embodiments, the sugar motifs of the two wings are identical to each other (symmetric gapmer). In certain embodiments, the sugar motif of the 5'-wing is different from the sugar motif of the 3'-wing (asymmetric gapmer).

In some embodiments, the gapmer comprises: (a) a 5' region consisting of 1-6 linked 5' region nucleosides; (b) a central region consisting of 6-10 linked central region nucleosides; and (c) a 3' region consisting of 1-6 linked 3' region nucleosides; wherein each of the nucleosides in the 5' region and the 3' region comprises a sugar moiety selected from 2'-MOE, LNA, and cEt modifications, and at least 6 of the nucleosides in the central region comprise a 2'-deoxy sugar moiety.

In some embodiments, the gapmer has a sugar motif (5' to 3') selected from: eekddddddddddkke, ekkddddddddddkke, kkkdyddddddddkkk, kkkddydddddddkkk, kkkdddyddddddkkk, kkkddddddddddkkk, or eeeeeddddddddddeeeee; wherein e represents a 2'-MOE sugar moiety, k represents a cEt sugar moiety, d represents a 2'-deoxy sugar moiety, and y represents a 2'-OMe sugar moiety.

In preferred embodiments, the gapmer has the sugar motif eeeeeeddddddddddeeeee (5' to 3'), wherein e represents a 2'-MOE sugar moiety and d represents a 2'-deoxy sugar moiety.

In some embodiments, said oligonucleotide is the sense or antisense strand of an RNAi agent. In such embodiments, the lipid-oligonucleotide conjugate preferably further comprises the antisense or sense strand of the RNAi agent, such that the sense and antisense strands pair to form a duplex region.

In some embodiments, the length of the duplex region is about 17 to about 23 base pairs. For example, suitable lengths for the duplex region are about 17 to about 22 base pairs, about 17 to about 21 base pairs, about 17 to about 20 base pairs, about 17 to about 19 base pairs, about 17 to about 18 base pairs, about 18 to about 23 base pairs, about 18 to about 22 base pairs, about 18 to about 21 base pairs, about 18 to about 20 base pairs, about 19 to 23 base pairs, about 19 to 22 base pairs, about 19 to 21 base pairs, about 20 to 23 base pairs, about 20 to 22 base pairs, or about 21 to 23 base pairs. In certain embodiments, the length of the duplex region is about 18 to about 21 base pairs. In other embodiments, the length of the duplex region is about 19 base pairs.

In some embodiments, the sense and antisense strands of the RNAi agent of the invention each independently have a length of about 17 to about 23 nucleotides, e.g., about 18 to about 23 nucleotides, about 19 to about 23 nucleotides, about 20 to about 23 nucleotides, about 21 to about 23 nucleotides, about 17 to about 22 nucleotides, about 17 to about 21 nucleotides, about 17 to about 20 nucleotides, about 17 to about 19 nucleotides, about 18 to about 22 nucleotides, about 18 to about 21 nucleotides, about 18 to about 20 nucleotides, about 19 to about 22 nucleotides, about 19 to about 21 nucleotides, or about 20 to about 22 nucleotides. In certain embodiments, the sense and antisense strands each independently have a length of about 17, about 18, about 19, about 20, about 21, about 22, or about 23 nucleotides.

In some embodiments, the sense strand and antisense strand are of identical length but form a duplex region shorter than the full length of the strands, such that the RNAi agent for inhibiting a target gene expression comprises two nucleotide overhangs. For example, in one embodiment, the RNAi agent for inhibiting a target gene expression comprises (i) a sense strand and an antisense strand each having a length of 21 nucleotides, (ii) a duplex region having a length of 19 base pairs, and (iii) a single-nucleotide overhang at each of 3'-end of the sense strand and 3'-end of the antisense strand. In another embodiment, the RNAi agent for inhibiting a target gene expression comprises (i) a sense strand and an antisense strand each having a length of 23 nucleotides, (ii) a duplex region having a length of 21 base pairs, and (iii) a single-nucleotide overhang at each of 3'-end of the sense strand and 3'-end of the antisense strand.

In other embodiments, the sense strand and antisense strand are of identical length and form a duplex region along their entire length, such that no nucleotide overhangs are present at either end of the duplex molecule. In one such embodiment, the RNAi agent for inhibiting a target gene expression is blunt-ended and comprises (i) a sense strand and an antisense strand each having a length of 21 nucleotides, and (ii) a duplex region having a length of 21 base pairs. In another such embodiment, the RNAi agent for inhibiting a target gene expression is blunt-ended and comprises (i) a sense strand and an antisense strand each having a length of 23 nucleotides, and (ii) a duplex region having a length of 23 base pairs. In another such embodiment, the RNAi agent for inhibiting a target gene expression is blunt-ended and comprises (i) a sense strand and an antisense strand each having a length of 19 nucleotides, and (ii) a duplex region having a length of 19 base pairs.

In other embodiments, the sense strand or antisense strand is longer than the other strand, and the two strands form a duplex region of length equal to that of the shorter strand, such that the RNAi agent for inhibiting a target gene expression comprises at least one nucleotide overhang. For example, in some embodiments, the sense strand is 1 to 4 nucleotides longer than the antisense strand, and the duplex region formed by both strands equals the length of the antisense strand, such that the sense strand forms an overhang comprising 1 to 4 unpaired nucleotides. In other embodiments, the antisense strand is 1 to 4 nucleotides longer than the sense strand, and the duplex region formed by both strands equals the length of the sense strand, such that the antisense strand forms an overhang comprising 1 to 4 unpaired nucleotides. In some embodiments, the nucleotide overhang has a length of 1, 2, 3, or 4 nucleotides. In a specific embodiment, the overhang comprises 2 nucleotides. In certain embodiments, the overhang comprises a single nucleotide.

The overhanging nucleotides may be ribonucleotides or modified nucleotides as described herein. In some embodiments, the overhanging nucleotides are 2'-modified nucleotides (e.g., 2'-fluoro-modified nucleotides, 2'-O-methyl-modified nucleotides) or combinations thereof. For example, in one embodiment, the overhanging nucleotides are deoxyribonucleotides, such as deoxythymidine. In another embodiment, the overhanging nucleotides are selected from: 2'-O-methyl-modified nucleotides, 2'-fluoro-modified nucleotides, 2'-methoxyethyl-modified nucleotides, abasic nucleotides, inverted abasic nucleotides, inverted nucleotides, or combinations thereof. In other embodiments, the overhang comprises a 5'-uridine-uridine-3' (5'-UU-3') dinucleotide. In such embodiments, the UU dinucleotide may comprise ribonucleotides or modified nucleotides, such as 2'-modified nucleotides. In other embodiments, the overhang comprises a 5'-deoxythymidine-deoxythymidine-3' (5'-dTdT-3') dinucleotide. When a nucleotide overhang is present on the antisense strand, nucleotides in the overhang may be complementary to the target gene sequence, may form mismatches with the target gene sequence, or may comprise other sequences (such as UU, TT, AA, GG, etc.).

The nucleotide overhang may be located at the 5'-end or 3'-end of either strand or both strands. For example, in one embodiment, the RNAi agent for inhibiting a target gene expression comprises nucleotide overhangs at both 5'-end and 3'-end of the antisense strand. In another embodiment, the RNAi agent for inhibiting a target gene expression comprises nucleotide overhangs at both 5'-end and 3'-end of the sense strand. In some embodiments, the RNAi agent for inhibiting a target gene expression comprises nucleotide overhangs at the 5'-end of the sense strand and the 5'-end of the antisense strand. In other embodiments, the RNAi agent for inhibiting a target gene expression comprises nucleotide overhangs at the 3'-end of the sense strand and the 3'-end of the antisense strand. In some embodiments, the RNAi agent for inhibiting a target gene expression comprises only a nucleotide overhang at the 5'-end of the sense strand. In some embodiments, the RNAi agent for inhibiting a target gene expression comprises only a nucleotide overhang at the 3'-end of the sense strand. In some embodiments, the RNAi agent for inhibiting a target gene expression comprises only a nucleotide overhang at the 3'-end of the antisense strand. In some embodiments, the RNAi agent for inhibiting a target gene expression comprises only a nucleotide overhang at the 5'-end of the antisense strand. In some embodiments, the RNAi agent for inhibiting a target gene expression comprises only a nucleotide overhang at the 5'-end of the sense strand.

The RNAi agent for inhibiting a target gene expression may comprise a nucleotide overhang at one end and a blunt end at the opposite end of the double-stranded RNA molecule. "Blunt end" means that the sense strand and antisense strand are fully base-paired at the molecular terminus, with no unpaired nucleotides extending beyond the duplex region. In some embodiments, the RNAi agent for inhibiting a target gene expression comprises a nucleotide overhang at the 3'-end of the sense strand, and blunt ends at the 5'-end of the sense strand and the 3'-end of the antisense strand. In other embodiments, the RNAi agent for inhibiting a target gene expression comprises a nucleotide overhang at the 3'-end of the antisense strand, and blunt ends at the 5'-end of the antisense strand and the 3'-end of the sense strand.

Specifically, by way of example, in one embodiment, the RNAi agent for inhibiting a target gene expression comprises: (i) a sense strand of 19 nucleotides in length, (ii) an antisense strand of 21 nucleotides in length, wherein the strands form a duplex region equal to the length of the sense strand. In another embodiment, the RNAi agent for inhibiting a target gene expression comprises: (i) a sense strand of 21 nucleotides in length, (ii) an antisense strand of 23 nucleotides in length, wherein the strands form a duplex region equal to the length of the sense strand.

In some embodiments, the RNAi agent that can form the lipid-oligonucleotide conjugate of the present invention comprises a sense strand and an antisense strand forming a duplex region, wherein the antisense strand is no more than 23 nucleotides in length, and the RNAi agent comprises one overhang and one blunt end, the overhang preferably having 2 unpaired nucleotides.

In some embodiments, the RNAi agent that can form the lipid-oligonucleotide conjugate of the present invention comprises a sense strand and an antisense strand forming a duplex region, wherein the antisense strand is no more than 23 nucleotides in length, and the RNAi agent comprises one overhang and one blunt end, the overhang preferably having 2 unpaired nucleotides, wherein the overhang is formed at the 3' end of the antisense strand, and the blunt end is formed at the 3' end of the sense strand and the 5' end of the antisense strand.

In some embodiments, the RNAi agent that can form the lipid-oligonucleotide conjugate of the present invention comprises a sense strand and an antisense strand forming a duplex region of 19 base pairs in length, wherein the antisense strand is no more than 23 nucleotides in length, and the RNAi agent comprises one overhang and one blunt end, the overhang preferably having 2 unpaired nucleotides, wherein the overhang is formed at the 3' end of the antisense strand, and the blunt end is formed at the 3' end of the sense strand and the 5' end of the antisense strand.

In some embodiments, the RNAi agent that can form the lipid-oligonucleotide conjugate of the present invention comprises a sense strand and an antisense strand forming a duplex region of 19 base pairs in length, wherein the antisense strand is no more than 23 nucleotides in length, the sense strand is 19 to 21 nucleotides in length, and the RNAi agent comprises one overhang and one blunt end, the overhang preferably having 2 unpaired nucleotides, wherein the overhang is formed at the 3' end of the antisense strand, and the blunt end is formed at the 3' end of the sense strand and the 5' end of the antisense strand.

In some embodiments, the RNAi agent that can form the lipid-oligonucleotide conjugate of the present invention comprises a sense strand and an antisense strand forming a duplex region of 19 base pairs in length, wherein the antisense strand is 21 nucleotides in length, the sense strand is 19 nucleotides in length, and the RNAi agent comprises one overhang and one blunt end, the overhang preferably having 2 unpaired nucleotides, wherein the overhang is formed at the 3' end of the antisense strand, and the blunt end is formed at the 3' end of the sense strand and the 5' end of the antisense strand.

In some embodiments, the RNAi agent of the present invention that can form the lipid-oligonucleotide conjugate comprises a sense strand and an antisense strand forming a duplex region of 19 base pairs in length, wherein the sense strand is 19 nucleotides in length, and the RNAi agent comprises one overhang and one blunt end, the overhang preferably having 2 unpaired nucleotides, wherein the overhang is formed at the 3' end of the antisense strand, and the blunt end is formed at the 3' end of the sense strand and the 5' end of the antisense strand.

In the present invention, the sense strand and/or antisense strand of said RNAi agent may comprise at least one modified nucleotide. In some embodiments, the sense strand and antisense strand of said RNAi agent each comprise at least one modified nucleotide. In some embodiments, each nucleotide of the sense strand and antisense strand of said RNAi agent is modified.

In any of the above embodiments, the modified nucleotide is independently selected from the group consisting of: 2'-deoxy-thymidine (dT) nucleotide, 2'-O-methyl-modified nucleotide (2'-OMe), 2'-fluoro-modified nucleotide (2'-F), 2'-deoxy-modified nucleotide, locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide (cEt), 2'-amino-modified nucleotide, 2'-O-allyl-modified nucleotide, 2'-C-alkyl-modified nucleotide, 2'-O-methoxyethyl-modified nucleotide (2'-MOE), abasic nucleotide, inverted abasic nucleotide, inverted nucleotide, morpholino nucleotide (MOP), phosphoramidate (PN), tetrahydropyran-modified nucleotide (THP), 1,5-anhydrohexitol-modified nucleotide (HNA), cyclohexenyl-modified nucleotide, nucleotide comprising a phosphorothioate group (PS), nucleotide comprising a methylphosphonate group, nucleotide comprising a 5'-phosphate, nucleotide comprising a 5'-phosphate mimic, cationic lipid-conjugated nucleotide, nucleotide comprising a 5'-vinylphosphonate (5'-VP), and combinations thereof.

In preferred embodiments, the modified nucleotides are independently selected from the group consisting of 2'-O-methyl-modified nucleotides, 2'-fluoro-modified nucleotides, nucleotides comprising phosphorothioate internucleoside linkages, and combinations thereof. In preferred embodiments, every nucleotide of the sense strand and/or the antisense strand of the RNAi agent is modified. In preferred embodiments, every nucleotide of the sense strand and/or the antisense strand of the RNAi agent is modified, and the modified nucleotides are independently selected from the group consisting of 2'-O-methyl-modified nucleotides, 2'-fluoro-modified nucleotides, nucleotides comprising phosphorothioate internucleoside linkages, and combinations thereof.

In preferred embodiments, each of the nucleotides in the sense strand and the antisense strand of the RNAi agent for inhibiting a target gene expression provided by the present invention is modified, and the modification is selected from the group consisting of STC(Alnylam), ESC(Alnylam), Advanced ESC(Alnylam), ESC+(Alnylam), AD1-3(Arrowhead), AD5(Arrowhead) and GalXC(Dicerna) (see, e.g., Hu B, Zhong L, Weng Y, et al. Therapeutic siRNA: state of the art. Signal Transduct Target Ther: 2020; 5(1):101. Published 2020 Jun 19. doi:10.1038/s41392-020-0207-x).

In preferred embodiments, the 5'-terminal nucleotide of the antisense strand of the RNAi agent for inhibiting a target gene expression provided by the present invention contains a phosphate or phosphate analog modification, preferably 5'-VP (see Parmar R, Willoughby JL, Liu J, et al. 5'-(E)-Vinylphosphonate: A Stable Phosphate Mimic Can Improve the RNAi Activity of siRNA-GalNAc Conjugates. Chembiochem. 2016;17(11):985-989).

In some embodiments, each of the nucleotides of the antisense strand of the RNAi agent is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

In some embodiments, each of the nucleotides of the antisense strand of the RNAi agent is modified, and in the 5' to 3' direction, the nucleotides at positions 2, 5, 7, 12, and 14 of the antisense strand are 2'-fluoro-modified nucleotides, while the nucleotides at remaining positions of the antisense strand are all 2'-methoxy-modified nucleotides.

In some embodiments, each of the nucleotides of the antisense strand of the RNAi agent is modified, and in the 5' to 3' direction, the nucleotides at positions 2, 5, 7, 14, and 16 of the antisense strand are 2'-fluoro-modified nucleotides, while the nucleotides at remaining positions of the antisense strand are all 2'-methoxy-modified nucleotides.

In some embodiments, each of the nucleotides of the antisense strand of the RNAi agent is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage. In preferable embodiments, the phosphorothioate internucleotide linkage is present at one or more of the following locations: (i) between nucleotide positions 1 and 2 at the 5'-end of the antisense strand; (ii) between nucleotide positions 2 and 3 at the 5'-end of the antisense strand; (iii) between nucleotide positions 1 and 2 at the 3'-end of the antisense strand; and (iv) between nucleotide positions 2 and 3 at the 3'-end of the antisense strand. In preferable embodiments, the phosphorothioate internucleotide linkage is present (i) between nucleotide positions 1 and 2 at the 5'-end of the antisense strand; (ii) between nucleotide positions 2 and 3 at the 5'-end of the antisense strand; (iii) between nucleotide positions 1 and 2 at the 3'-end of the antisense strand; and (iv) between nucleotide positions 2 and 3 at the 3'-end of the antisense strand.

In some embodiments, each of the nucleotides of the sense and antisense strands of the RNAi agent is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides.

For example, each of the nucleotides of the sense and antisense strands of the RNAi agent is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides.

For example, each of the nucleotides of the sense and antisense strands of the RNAi agent is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides.

For example, each of the nucleotides of the sense and antisense strands of the RNAi agent is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides.

For example, each of the nucleotides of the sense and antisense strands of the RNAi agent is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7, 9, and 11 of the sense strand are 2'-fluoro-modified nucleotides, while the nucleotides at remaining positions of the sense strand are all 2'-methoxy-modified nucleotides.

In preferred embodiments, the sense strand has at least one phosphorothioate internucleoside linkage. In preferred embodiments, the phosphorothioate internucleoside linkage is present in one or more of the following positions: (i) between nucleotide position 1 and nucleotide position 2 at the 5' end of the sense strand; (ii) between nucleotide position 2 and nucleotide position 3 at the 5' end of the sense strand; (iii) between nucleotide position 1 and nucleotide position 2 at the 3' end of the sense strand; and (iv) between nucleotide position 2 and nucleotide position 3 at the 3' end of the sense strand. In more preferred embodiments, the phosphorothioate internucleoside linkages are present between nucleotide position 1 and nucleotide position 2 at the 5' end of the sense strand; and between nucleotide position 2 and nucleotide position 3 at the 5' end of the sense strand. In more preferred embodiments, the phosphorothioate internucleoside linkages are present between nucleotide position 1 and nucleotide position 2 at the 5' end of the sense strand; between nucleotide position 2 and nucleotide position 3 at the 5' end of the sense strand; between nucleotide position 1 and nucleotide position 2 at the 3' end of the sense strand; and between nucleotide position 2 and nucleotide position 3 at the 3' end of the sense strand.

In preferred embodiments, the antisense strand has at least one phosphorothioate internucleoside linkage. Preferably, the phosphorothioate internucleoside linkage is present in one or more of the following: between nucleotide position 1 and nucleotide position 2 at the 5' end of the antisense strand; between nucleotide position 2 and nucleotide position 3 at the 5' end of the antisense strand; between nucleotide position 1 and nucleotide position 2 at the 3' end of the antisense strand; and between nucleotide position 2 and nucleotide position 3 at the 3' end of the antisense strand.

Accordingly, in some embodiments, each of the nucleotides of the sense and antisense strands of the RNAi agent is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage, wherein the phosphorothioate internucleotide linkage is present at one or more of the following locations: between nucleotide positions 1 and 2 at the 5'-end of the antisense strand; between nucleotide positions 2 and 3 at the 5'-end of the antisense strand; between nucleotide positions 1 and 2 at the 3'-end of the antisense strand; and between nucleotide positions 2 and 3 at the 3'-end of the antisense strand; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides; and the sense strand comprises at least one phosphorothioate internucleotide linkage, wherein the phosphorothioate internucleotide linkage is present between nucleotide positions 1 and 2 at the 5'-end of the sense strand and/or between nucleotide positions 2 and 3 at the 5'-end of the sense strand.

In some embodiments, each of the nucleotides of the sense and antisense strands of the RNAi agent is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage, wherein the phosphorothioate internucleotide linkage is present: between nucleotide positions 1 and 2 at the 5'-end of the antisense strand; between nucleotide positions 2 and 3 at the 5'-end of the antisense strand; between nucleotide positions 1 and 2 at the 3'-end of the antisense strand; and between nucleotide positions 2 and 3 at the 3'-end of the antisense strand; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides; and the sense strand comprises at least one phosphorothioate internucleotide linkage, wherein the phosphorothioate internucleotide linkage is present between nucleotide positions 1 and 2 at the 5'-end of the sense strand and between nucleotide positions 2 and 3 at the 5'-end of the sense strand.

### Intermediate Compounds, Ligand Moieties, and Uses

Another aspect of the present invention provides intermediate compounds selected from compounds of the following structural formulae:

| | |
|---|---|
| **1-5** | 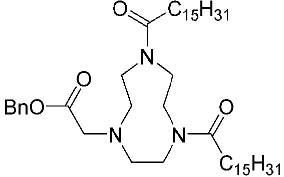 |
| **1-6** | 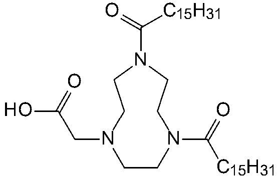 |
| **1-7** | 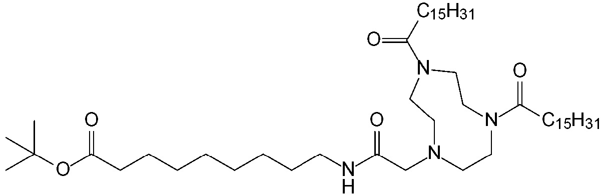 |
| **1-8** | 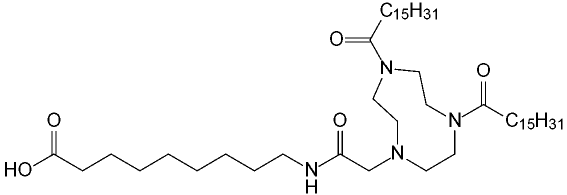 |
| **LL1p** | 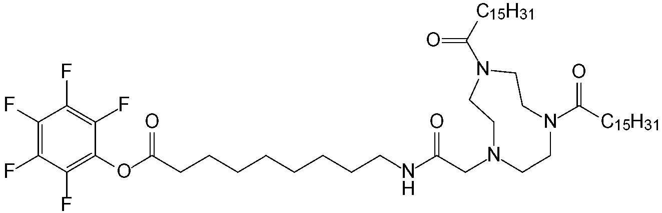 |
| **2-1** | 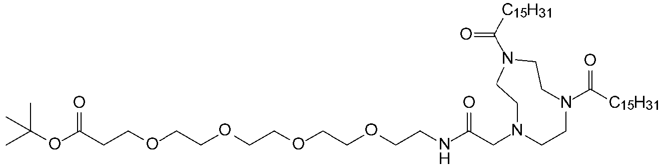 |
| **2-2** | 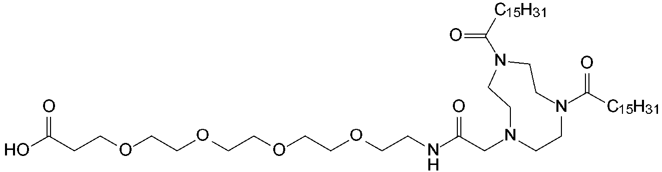 |
| **LL2p** | 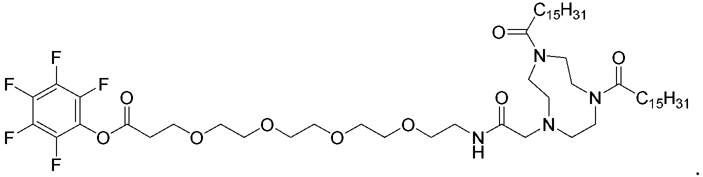 |

The present invention also provides a ligand moiety represented by Formula II for forming a lipid-oligonucleotide conjugate: 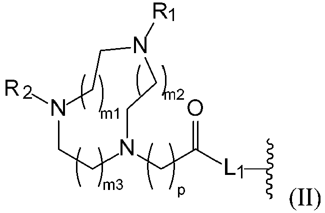 wherein,
at least one of R1 and R2 is selected from Ra, -C(O)Ra, -C(O)NHRa, and C(O)ORa, wherein Ra is optionally substituted C5-30 alkyl or alkenyl, and
the other of R1 and R2 is selected from H, Rb, -C(O)Rb, -C(O)NHRb, and C(O)ORb, wherein Rb is selected from optionally substituted C1-4 alkyl or alkenyl, optionally substituted C5-30 alkyl or alkenyl, optionally substituted C2-10 alkynyl, optionally substituted C1-10 alkoxy, optionally substituted C3-10 cycloalkyl, optionally substituted 6- to 14-membered aryl, optionally substituted 5- to 18-membered heteroaryl, optionally substituted 5- to 14-membered heterocyclyl;
said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5- to 18-membered heteroaryl, 5- to 14-membered heterocyclyl, halogen, hydroxyl, amino, guanidino, carboxyl, cyano, nitro, C1-3 alkylthio, and mercapto;
m1, m2, and m3 are each independently an integer of 1 or 2;
p is 0, 1, or 2; and 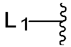
represents a linker, and the wavy line represents the position of attachment to the remainder of the lipid-oligonucleotide conjugate.

In some embodiments, one of R1 and R2 is -C(O)Ra, wherein Ra is optionally substituted C5-30 alkyl or alkenyl, and the other of R1 and R2 is H or -C(O)C1-4 alkyl or alkenyl.

In some embodiments, R1 is -C(O)Ra, wherein Ra is optionally substituted C5-30 alkyl or alkenyl, and R2 is H or -C(O)C1-4 alkyl or alkenyl.

In some embodiments, R2 is -C(O)Ra, wherein Ra is optionally substituted C5-30 alkyl or alkenyl, and R1 is H or -C(O)C1-4 alkyl or alkenyl.

In preferred embodiments, Ra is C5-30 alkyl or alkenyl. In preferred embodiments, Ra is C12-24 alkyl or alkenyl. In preferred embodiments, Ra is C14-22 alkyl or alkenyl. In preferred embodiments, Ra is C14, C15, C16, C18, C20, or C22 alkyl, monounsaturated alkenyl, or polyunsaturated alkenyl.

For example, Ra is selected from C5 alkyl, C6 alkyl, C7 alkyl, C8 alkyl, C9 alkyl, C10 alkyl, C11 alkyl, C12 alkyl, C13 alkyl, C14 alkyl, C15 alkyl, C16 alkyl, C17 alkyl, C18 alkyl, C19 alkyl, C20 alkyl, C21 alkyl, C22 alkyl, C23 alkyl, C24 alkyl, C25 alkyl, C26 alkyl, C27 alkyl, C28 alkyl, C29 alkyl, C30 alkyl, C5 monounsaturated alkenyl, C6 monounsaturated alkenyl, C7 monounsaturated alkenyl, C8 monounsaturated alkenyl, C9 monounsaturated alkenyl, C10 monounsaturated alkenyl, C11 monounsaturated alkenyl, C12 monounsaturated alkenyl, C13 monounsaturated alkenyl, C14 monounsaturated alkenyl, C15 monounsaturated alkenyl, C16 monounsaturated alkenyl, C17 monounsaturated alkenyl, C18 monounsaturated alkenyl, C19 monounsaturated alkenyl, C20 monounsaturated alkenyl, C21 monounsaturated alkenyl, C22 monounsaturated alkenyl, C23 monounsaturated alkenyl, C24 monounsaturated alkenyl, C25 monounsaturated alkenyl, C26 monounsaturated alkenyl, C27 monounsaturated alkenyl, C28 monounsaturated alkenyl, C29 monounsaturated alkenyl, C30 monounsaturated alkenyl, C5 polyunsaturated alkenyl, C6 polyunsaturated alkenyl, C7 polyunsaturated alkenyl, C8 polyunsaturated alkenyl, C9 polyunsaturated alkenyl, C10 polyunsaturated alkenyl, C11 polyunsaturated alkenyl, C12 polyunsaturated alkenyl, C13 polyunsaturated alkenyl, C14 polyunsaturated alkenyl, C15 polyunsaturated alkenyl, C16 polyunsaturated alkenyl, C17 polyunsaturated alkenyl, C18 polyunsaturated alkenyl, C19 polyunsaturated alkenyl, C20 polyunsaturated alkenyl, C21 polyunsaturated alkenyl, C22 polyunsaturated alkenyl, C23 polyunsaturated alkenyl, C24 polyunsaturated alkenyl, C25 polyunsaturated alkenyl, C26 polyunsaturated alkenyl, C27 polyunsaturated alkenyl, C28 polyunsaturated alkenyl, C29 polyunsaturated alkenyl, and C30 polyunsaturated alkenyl.

In preferred embodiments, both of R1 and R2 are and independently are -C(O)Ra, wherein Ra is optionally substituted C5-30 alkyl or alkenyl. In preferred embodiments, both of R1 and R2 are -C(O)Ra, and Ra is optionally substituted C5-30 alkyl or alkenyl. In preferred embodiments, R1 and R2 are the same and Ra is C5-30 alkyl or alkenyl. In preferred embodiments, R1 and R2 are the same and Ra is C12-24 alkyl or alkenyl. In preferred embodiments, R1 and R2 are the same and Ra is C14-22 alkyl or alkenyl. In preferred embodiments, R1 and R2 are the same and Ra is C14, C15, C16, C18, C20, or C22 alkyl, monounsaturated alkenyl, or polyunsaturated alkenyl.

In preferred embodiments, R1 and R2 are the same and Ra is selected from C5 alkyl, C6 alkyl, C7 alkyl, C8 alkyl, C9 alkyl, C10 alkyl, C11 alkyl, C12 alkyl, C13 alkyl, C14 alkyl, C15 alkyl, C16 alkyl, C17 alkyl, C18 alkyl, C19 alkyl, C20 alkyl, C21 alkyl, C22 alkyl, C23 alkyl, C24 alkyl, C25 alkyl, C26 alkyl, C27 alkyl, C28 alkyl, C29 alkyl, C30 alkyl, C5 monounsaturated alkenyl, C6 monounsaturated alkenyl, C7 monounsaturated alkenyl, C8 monounsaturated alkenyl, C9 monounsaturated alkenyl, C10 monounsaturated alkenyl, C11 monounsaturated alkenyl, C12 monounsaturated alkenyl, C13 monounsaturated alkenyl, C14 monounsaturated alkenyl, C15 monounsaturated alkenyl, C16 monounsaturated alkenyl, C17 monounsaturated alkenyl, C18 monounsaturated alkenyl, C19 monounsaturated alkenyl, C20 monounsaturated alkenyl, C21 monounsaturated alkenyl, C22 monounsaturated alkenyl, C23 monounsaturated alkenyl, C24 monounsaturated alkenyl, C25 monounsaturated alkenyl, C26 monounsaturated alkenyl, C27 monounsaturated alkenyl, C28 monounsaturated alkenyl, C29 monounsaturated alkenyl, C30 monounsaturated alkenyl, C5 polyunsaturated alkenyl, C6 polyunsaturated alkenyl, C7 polyunsaturated alkenyl, C8 polyunsaturated alkenyl, C9 polyunsaturated alkenyl, C10 polyunsaturated alkenyl, C11 polyunsaturated alkenyl, C12 polyunsaturated alkenyl, C13 polyunsaturated alkenyl, C14 polyunsaturated alkenyl, C15 polyunsaturated alkenyl, C16 polyunsaturated alkenyl, C17 polyunsaturated alkenyl, C18 polyunsaturated alkenyl, C19 polyunsaturated alkenyl, C20 polyunsaturated alkenyl, C21 polyunsaturated alkenyl, C22 polyunsaturated alkenyl, C23 polyunsaturated alkenyl, C24 polyunsaturated alkenyl, C25 polyunsaturated alkenyl, C26 polyunsaturated alkenyl, C27 polyunsaturated alkenyl, C28 polyunsaturated alkenyl, C29 polyunsaturated alkenyl, and C30 polyunsaturated alkenyl.

In some embodiments, m1, m2, and m3 are all 1. In some embodiments, two of m1, m2, and m3 are 1 and the other is 2. In some embodiments, m1 and m2 are 1, and m3 is 2. In some embodiments, m1 and m3 are 1, and m2 is 2. In some embodiments, m2 and m3 are 1, and m1 is 2.

In some embodiments, p is 2. In some embodiments, p is 0. In preferred embodiments, p is 1.

In some embodiments, the oligonucleotide is an antisense oligonucleotide or the sense strand or antisense strand of an RNAi agent. In some embodiments, the sense strand or antisense strand of said RNAi agent pairs with its antisense strand or sense strand to form a duplex. In preferred embodiments, said RNAi agent is siRNA.

In preferred embodiments, the oligonucleotide is an antisense oligonucleotide (ASO). In preferred embodiments, the oligonucleotide is the sense strand or antisense strand of siRNA. In preferred embodiments, the oligonucleotide is the sense strand of siRNA. In preferred embodiments, the sense strand or antisense strand of said siRNA pairs with its antisense strand or sense strand to form a duplex.

In some embodiments, wherein L1 is 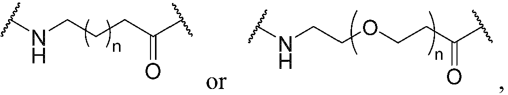 wherein each n is independently an integer from 1 to 10, preferably from 2 to 8, more preferably from 4 to 6; and the right side of the formula is connected to the remainder of the lipid-oligonucleotide conjugate.

In specific embodiments, the ligand has the structure of: 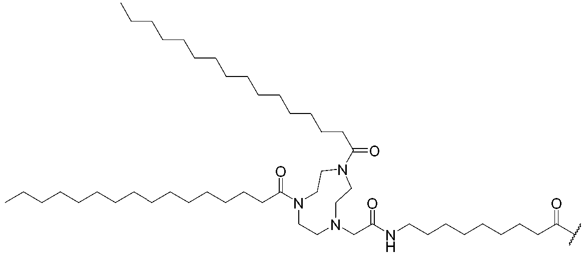 or

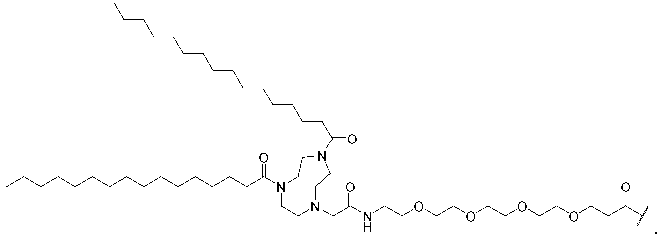

Another aspect of the present invention provides use of a ligand moiety represented by Formula III in the preparation of a lipid-oligonucleotide conjugate, 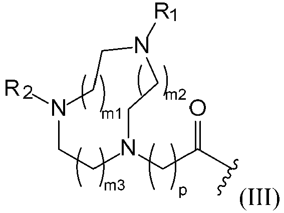 wherein,
at least one of R1 and R2 is selected from Ra, -C(O)Ra, -C(O)NHRa, and C(O)ORa, wherein Ra is optionally substituted C5-30 alkyl or alkenyl, and
the other of R1 and R2 is selected from H, Rb, -C(O)Rb, -C(O)NHRb, and C(O)ORb, wherein Rb is selected from optionally substituted C1-4 alkyl or alkenyl, optionally substituted C5-30 alkyl or alkenyl, optionally substituted C2-10 alkynyl, optionally substituted C1-10 alkoxy, optionally substituted C3-10 cycloalkyl, optionally substituted 6- to 14-membered aryl, optionally substituted 5- to 18-membered heteroaryl, optionally substituted 5- to 14-membered heterocyclyl;
said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5- to 18-membered heteroaryl, 5- to 14-membered heterocyclyl, halogen, hydroxyl, amino, guanidino, carboxyl, cyano, nitro, C1-3 alkylthio, and mercapto;
m1, m2, and m3 are each independently an integer of 1 or 2;
p is 0, 1, or 2; and
the wavy line represents the position of attachment to the remainder of the lipid-oligonucleotide conjugate.

In some embodiments, one of R1 and R2 is -C(O)Ra, wherein Ra is optionally substituted C5-30 alkyl or alkenyl, and the other of R1 and R2 is H or -C(O)C1-4 alkyl or alkenyl.

In some embodiments, R1 is -C(O)Ra, wherein Ra is optionally substituted C5-30 alkyl or alkenyl, and R2 is H or -C(O)C1-4 alkyl or alkenyl.

In some embodiments, R2 is -C(O)Ra, wherein Ra is optionally substituted C5-30 alkyl or alkenyl, and R1 is H or -C(O)C1-4 alkyl or alkenyl.

In preferred embodiments, Ra is C5-30 alkyl or alkenyl. In preferred embodiments, Ra is C12-24 alkyl or alkenyl. In preferred embodiments, Ra is C14-22 alkyl or alkenyl. In preferred embodiments, Ra is C14, C15, C16, C18, C20, or C22 alkyl, monounsaturated alkenyl, or polyunsaturated alkenyl.

For example, Ra is selected from C5 alkyl, C6 alkyl, C7 alkyl, C8 alkyl, C9 alkyl, C10 alkyl, C11 alkyl, C12 alkyl, C13 alkyl, C14 alkyl, C15 alkyl, C16 alkyl, C17 alkyl, C18 alkyl, C19 alkyl, C20 alkyl, C21 alkyl, C22 alkyl, C23 alkyl, C24 alkyl, C25 alkyl, C26 alkyl, C27 alkyl, C28 alkyl, C29 alkyl, C30 alkyl, C5 monounsaturated alkenyl, C6 monounsaturated alkenyl, C7 monounsaturated alkenyl, C8 monounsaturated alkenyl, C9 monounsaturated alkenyl, C10 monounsaturated alkenyl, C11 monounsaturated alkenyl, C12 monounsaturated alkenyl, C13 monounsaturated alkenyl, C14 monounsaturated alkenyl, C15 monounsaturated alkenyl, C16 monounsaturated alkenyl, C17 monounsaturated alkenyl, C18 monounsaturated alkenyl, C19 monounsaturated alkenyl, C20 monounsaturated alkenyl, C21 monounsaturated alkenyl, C22 monounsaturated alkenyl, C23 monounsaturated alkenyl, C24 monounsaturated alkenyl, C25 monounsaturated alkenyl, C26 monounsaturated alkenyl, C27 monounsaturated alkenyl, C28 monounsaturated alkenyl, C29 monounsaturated alkenyl, C30 monounsaturated alkenyl, C5 polyunsaturated alkenyl, C6 polyunsaturated alkenyl, C7 polyunsaturated alkenyl, C8 polyunsaturated alkenyl, C9 polyunsaturated alkenyl, C10 polyunsaturated alkenyl, C11 polyunsaturated alkenyl, C12 polyunsaturated alkenyl, C13 polyunsaturated alkenyl, C14 polyunsaturated alkenyl, C15 polyunsaturated alkenyl, C16 polyunsaturated alkenyl, C17 polyunsaturated alkenyl, C18 polyunsaturated alkenyl, C19 polyunsaturated alkenyl, C20 polyunsaturated alkenyl, C21 polyunsaturated alkenyl, C22 polyunsaturated alkenyl, C23 polyunsaturated alkenyl, C24 polyunsaturated alkenyl, C25 polyunsaturated alkenyl, C26 polyunsaturated alkenyl, C27 polyunsaturated alkenyl, C28 polyunsaturated alkenyl, C29 polyunsaturated alkenyl, and C30 polyunsaturated alkenyl.

In preferred embodiments, both of R1 and R2 are and independently are -C(O)Ra, wherein Ra is optionally substituted C5-30 alkyl or alkenyl. In preferred embodiments, both of R1 and R2 are -C(O)Ra, and Ra is optionally substituted C5-30 alkyl or alkenyl. In preferred embodiments, R1 and R2 are the same and Ra is C5-30 alkyl or alkenyl. In preferred embodiments, R1 and R2 are the same and Ra is C12-24 alkyl or alkenyl. In preferred embodiments, R1 and R2 are the same and Ra is C14-22 alkyl or alkenyl. In preferred embodiments, R1 and R2 are the same and Ra is C14, C15, C16, C18, C20, or C22 alkyl, monounsaturated alkenyl, or polyunsaturated alkenyl.

In preferred embodiments, R1 and R2 are the same and Ra is selected from C5 alkyl, C6 alkyl, C7 alkyl, C8 alkyl, C9 alkyl, C10 alkyl, C11 alkyl, C12 alkyl, C13 alkyl, C14 alkyl, C15 alkyl, C16 alkyl, C17 alkyl, C18 alkyl, C19 alkyl, C20 alkyl, C21 alkyl, C22 alkyl, C23 alkyl, C24 alkyl, C25 alkyl, C26 alkyl, C27 alkyl, C28 alkyl, C29 alkyl, C30 alkyl, C5 monounsaturated alkenyl, C6 monounsaturated alkenyl, C7 monounsaturated alkenyl, C8 monounsaturated alkenyl, C9 monounsaturated alkenyl, C10 monounsaturated alkenyl, C11 monounsaturated alkenyl, C12 monounsaturated alkenyl, C13 monounsaturated alkenyl, C14 monounsaturated alkenyl, C15 monounsaturated alkenyl, C16 monounsaturated alkenyl, C17 monounsaturated alkenyl, C18 monounsaturated alkenyl, C19 monounsaturated alkenyl, C20 monounsaturated alkenyl, C21 monounsaturated alkenyl, C22 monounsaturated alkenyl, C23 monounsaturated alkenyl, C24 monounsaturated alkenyl, C25 monounsaturated alkenyl, C26 monounsaturated alkenyl, C27 monounsaturated alkenyl, C28 monounsaturated alkenyl, C29 monounsaturated alkenyl, C30 monounsaturated alkenyl, C5 polyunsaturated alkenyl, C6 polyunsaturated alkenyl, C7 polyunsaturated alkenyl, C8 polyunsaturated alkenyl, C9 polyunsaturated alkenyl, C10 polyunsaturated alkenyl, C11 polyunsaturated alkenyl, C12 polyunsaturated alkenyl, C13 polyunsaturated alkenyl, C14 polyunsaturated alkenyl, C15 polyunsaturated alkenyl, C16 polyunsaturated alkenyl, C17 polyunsaturated alkenyl, C18 polyunsaturated alkenyl, C19 polyunsaturated alkenyl, C20 polyunsaturated alkenyl, C21 polyunsaturated alkenyl, C22 polyunsaturated alkenyl, C23 polyunsaturated alkenyl, C24 polyunsaturated alkenyl, C25 polyunsaturated alkenyl, C26 polyunsaturated alkenyl, C27 polyunsaturated alkenyl, C28 polyunsaturated alkenyl, C29 polyunsaturated alkenyl, and C30 polyunsaturated alkenyl.

In some embodiments, m1, m2, and m3 are all 1. In some embodiments, two of m1, m2, and m3 are 1 and the other is 2. In some embodiments, m1 and m2 are 1, and m3 is 2. In some embodiments, m1 and m3 are 1, and m2 is 2. In some embodiments, m2 and m3 are 1, and m1 is 2.

In some embodiments, p is 2. In some embodiments, p is 0. In preferred embodiments, p is 1.

In some embodiments, the oligonucleotide is an antisense oligonucleotide or the sense strand or antisense strand of an RNAi agent. In some embodiments, the sense strand or antisense strand of said RNAi agent pairs with its antisense strand or sense strand to form a duplex. In preferred embodiments, said RNAi agent is siRNA.

In preferred embodiments, the oligonucleotide is an antisense oligonucleotide (ASO). In preferred embodiments, the oligonucleotide is the sense strand or antisense strand of siRNA. In preferred embodiments, the oligonucleotide is the sense strand of siRNA. In preferred embodiments, the sense strand or antisense strand of said siRNA pairs with its antisense strand or sense strand to form a duplex.

### Pharmaceutical Compositions, Therapeutic Methods, and Uses

The present invention also includes pharmaceutical compositions or formulations comprising the lipid-oligonucleotide conjugates described herein and a pharmaceutically acceptable carrier, excipient, or diluent. Such compositions and formulations may be used to reduce expression of a target gene in a patient in need thereof. In the context of clinical applications, the pharmaceutical compositions and formulations will be prepared in a form suitable for the intended application. Typically, this will require preparing compositions that are substantially free of pyrogens and other impurities that may be harmful to humans or animals.

The ingredients and methods for formulating pharmaceutical compositions depend on a number of criteria, including but not limited to the route of administration, the type and extent of the disease or condition to be treated, or the dose to be administered. In some embodiments, pharmaceutical compositions are formulated based on the intended delivery route. For example, in certain embodiments, pharmaceutical compositions are formulated for parenteral delivery. Parenteral administration forms include intravenous, intraarterial, subcutaneous, intrathecal, intraperitoneal, or intramuscular injection or infusion. In one embodiment, the pharmaceutical composition is formulated for intravenous delivery. In such embodiments, the pharmaceutical composition may include a lipid-based delivery vehicle. In another embodiment, the pharmaceutical composition is formulated for subcutaneous delivery.

In embodiments, the lipid-oligonucleotide conjugate is preferably delivered to a specific tissue in the body after administration. In embodiments, the lipid-oligonucleotide conjugate is delivered to cardiac tissue after administration, for example. In embodiments, the lipid-oligonucleotide conjugate is delivered to splenic tissue after administration, for example. In embodiments, the lipid-oligonucleotide conjugate is delivered to renal tissue after administration, for example. In embodiments, the lipid-oligonucleotide conjugate is delivered to skin tissue after administration, for example. In embodiments, the lipid-oligonucleotide conjugate is delivered to muscle tissue after administration, for example. In embodiments, the lipid-oligonucleotide conjugate is delivered to lung tissue after administration, for example. In embodiments, the lipid-oligonucleotide conjugate is delivered to adipose tissue after administration, for example. In embodiments, the lipid-oligonucleotide conjugate is delivered to brain tissue after administration, for example.

In some embodiments, the pharmaceutical composition comprises a therapeutically or prophylactically effective amount of a lipid-oligonucleotide conjugate described herein. An "effective amount" refers to an amount sufficient to produce a beneficial or desired clinical result. In some embodiments, an effective amount is an amount sufficient to reduce target gene expression in a particular tissue or cell type (e.g., liver or hepatocytes) of a patient. The effective amount of a lipid-oligonucleotide conjugate of the present invention may range from about 0.01 mg/kg body weight to about 100 mg/kg body weight, and may be administered daily, weekly, monthly, or at longer intervals. The precise determination of the specific effective dosage and dosing frequency may be based on several factors, including the size, age, and general condition of the patient, the type of disease being treated (e.g., myocardial infarction, coronary artery disease, peripheral artery disease, stroke), the particular lipid-oligonucleotide conjugate used, and the route of administration.

Administration of the pharmaceutical compositions of the present invention may be via any common route, as long as the target tissue is accessible via that route. These routes include, but are not limited to, parenteral (e.g., subcutaneous, intramuscular, intraperitoneal, or intravenous), oral, nasal, buccal, intradermal, transdermal, and sublingual routes. In some embodiments, the pharmaceutical composition is administered parenterally. For example, in certain embodiments, the pharmaceutical composition is administered intravenously. In other embodiments, the pharmaceutical composition is administered subcutaneously. In other embodiments, the pharmaceutical composition is administered pulmonarily or intranasally.

Pharmaceutical compositions suitable for injectable use include, for example, sterile aqueous solutions or dispersions and sterile powders for the extemporaneous preparation of sterile injectable solutions or dispersions. Generally, these preparations are sterile and fluid to the extent that they are easily injectable. The preparations should be stable under the conditions of manufacture and storage and should be preserved against the contaminating action of microorganisms such as bacteria and fungi. Suitable solvents or dispersion media may include, for example, water, ethanol, polyol (e.g., glycerol, propylene glycol, and liquid polyethylene glycol, and the like), suitable mixtures thereof, and vegetable oils. Proper fluidity can be maintained, for example, by the use of a coating such as lecithin, by the maintenance of the required particle size in the case of dispersion, and by the use of surfactants. The action of microorganisms can be prevented by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of injectable compositions can be brought about by the use in the compositions of agents delaying absorption, for example, aluminum monostearate and gelatin.

Sterile injectable solutions can be prepared by incorporating the active compound in the required amount in the appropriate solvent with any of the other ingredients, for example, those enumerated above, followed by filter sterilization. Generally, dispersions are prepared by incorporating the various sterilized active ingredients into a sterile vehicle that contains a basic dispersion medium and the required other ingredients, for example, as described above. In the case of sterile powders for the preparation of sterile injectable solutions, preferred methods of preparation include vacuum drying and freeze-drying techniques that yield a powder of the active ingredient plus any additional desired ingredient from a previously sterile-filtered solution thereof.

The compositions of the present invention may generally be formulated in neutral or salt form. Pharmaceutically acceptable salts include, for example, acid addition salts (formed with free amino groups) derived from inorganic acids (such as hydrochloric or phosphoric acid) or organic acids (such as acetic, oxalic, tartaric, mandelic acid, and the like). Salts formed with free carboxyl groups may also be derived from inorganic bases (e.g., sodium, potassium, ammonium, calcium, or iron oxide) or organic bases (e.g., isopropylamine, trimethylamine, histidine, procaine, and the like). In some embodiments, the lipid-oligonucleotide conjugates of the present invention are formulated as sodium salts.

For example, for parenteral administration in aqueous solution, the solution is generally appropriately buffered, and the liquid diluent is first rendered isotonic with, for example, sufficient saline or glucose. Such aqueous solutions may be used, for example, for intravenous, intramuscular, subcutaneous, and intraperitoneal administration. Preferably, sterile aqueous media are used. For example, a single dose may be dissolved in 1 ml of isotonic NaCl solution and added to 1000 ml of subcutaneous infusion fluid or injected at the proposed infusion site. For human administration, the preparation should meet sterility, pyrogenicity, general safety, and purity standards as required by local FDA standards. In certain embodiments, the pharmaceutical composition of the present invention comprises or consists of a sterile saline solution and the lipid-oligonucleotide conjugate described herein. In other embodiments, the pharmaceutical composition of the present invention comprises or consists of the lipid-oligonucleotide conjugate described herein and sterile water (e.g., water for injection, WFI). In other embodiments, the pharmaceutical composition of the present invention comprises or consists of the lipid-oligonucleotide conjugate described herein and phosphate-buffered saline (PBS).

In some embodiments, the pharmaceutical compositions of the present invention are packaged with or stored within an administration device. Devices for injectable formulations include, but are not limited to, injection ports, prefilled syringes, auto-injectors, infusion pumps, in vivo injectors, and injection pens. Devices for nebulized or powder formulations include, but are not limited to, inhalers, insufflators, aspirators, and the like. Thus, the present invention includes administration devices containing the pharmaceutical compositions of the present invention for treating or preventing one or more diseases or conditions described herein.

In some embodiments of the present invention, the target gene that can be targeted by the oligonucleotide in the lipid-oligonucleotide conjugate may be selected from the group consisting of: SOD1, MALAT1, apolipoprotein B, apolipoprotein C III, endothelial lipase, p53, clusterin, signal transducer and activator of transcription 3 (STAT3), mothers against decapentaplegic homolog 7 (SMAD7), intercellular adhesion molecule 1 (CD54), dystrophin (e.g., myotonic dystrophy protein kinase (DMPK)), transthyretin (TTR), huntingtin (HTT), microRNA-122 (miR-122).

In certain embodiments, expression of the target gene in a cell is reduced by at least 40%, at least 45%, or at least 50% by the lipid-oligonucleotide conjugate of the present invention. In some embodiments, expression of the target gene in a cell is reduced by at least 60%, at least 65%, at least 70%, at least 75%, at least 80%, or at least 85% by the lipid-oligonucleotide conjugate of the present invention. In other embodiments, expression of the target gene in a cell is reduced by about 90% or more, e.g., 91%, 92%, 93%, 94%, 95%, 96%, 97%, 98%, 99% or more. The percentage reduction in target gene expression can be measured by any of the methods described herein and other methods known in the art.

In certain embodiments, the present invention provides a method for reducing target gene expression in a patient in need thereof, comprising administering to the patient any of the lipid-oligonucleotide conjugates described herein. Preferably, the level of expression of the target gene in the patient's cells after administration of the lipid-oligonucleotide conjugate is reduced compared to the level of target gene expression in a patient not receiving the lipid-oligonucleotide conjugate, or compared to the level of target gene expression in the patient prior to administration of the lipid-oligonucleotide conjugate. In some embodiments, after administration of the lipid-oligonucleotide conjugate of the present invention, expression of the target gene in the patient is reduced by at least 30%, at least 35%, at least 40%, at least 45%, at least 50%, at least 55%, at least 60%, at least 65%, at least 70%, at least 75%, at least 80%, at least 85%, or at least 90%, e.g., 91%, 92%, 93%, 94%, 95%, 96%, 97%, 98%, or 99%. The percentage reduction in target gene expression can be measured by any of the methods described herein and other methods known in the art. In certain embodiments, the percentage reduction in target gene expression is determined by assessing target gene protein levels in serum or plasma of the patient according to the methods described herein.

In certain other embodiments, the patient in need of reducing target gene expression is a patient with elevated circulating target gene levels. Accordingly, in some embodiments, the present invention provides a method of reducing serum or plasma levels of target gene protein in a patient in need thereof by administering to the patient any of the lipid-oligonucleotide conjugates described herein. In some embodiments, the present invention includes use of any of the lipid-oligonucleotide conjugates described herein in the preparation of a medicament for reducing serum or plasma levels of target gene protein in a patient in need thereof. In other embodiments, the present invention provides a target gene-targeting lipid-oligonucleotide conjugate for use in a method of reducing serum or plasma levels of target gene protein in a patient in need thereof.

The present invention also provides use of the lipid-oligonucleotide conjugates, intermediate compounds, or ligand moieties described herein in the preparation of lipid-oligonucleotide conjugates.

### Examples

### Example 1. Synthesis of LL1p

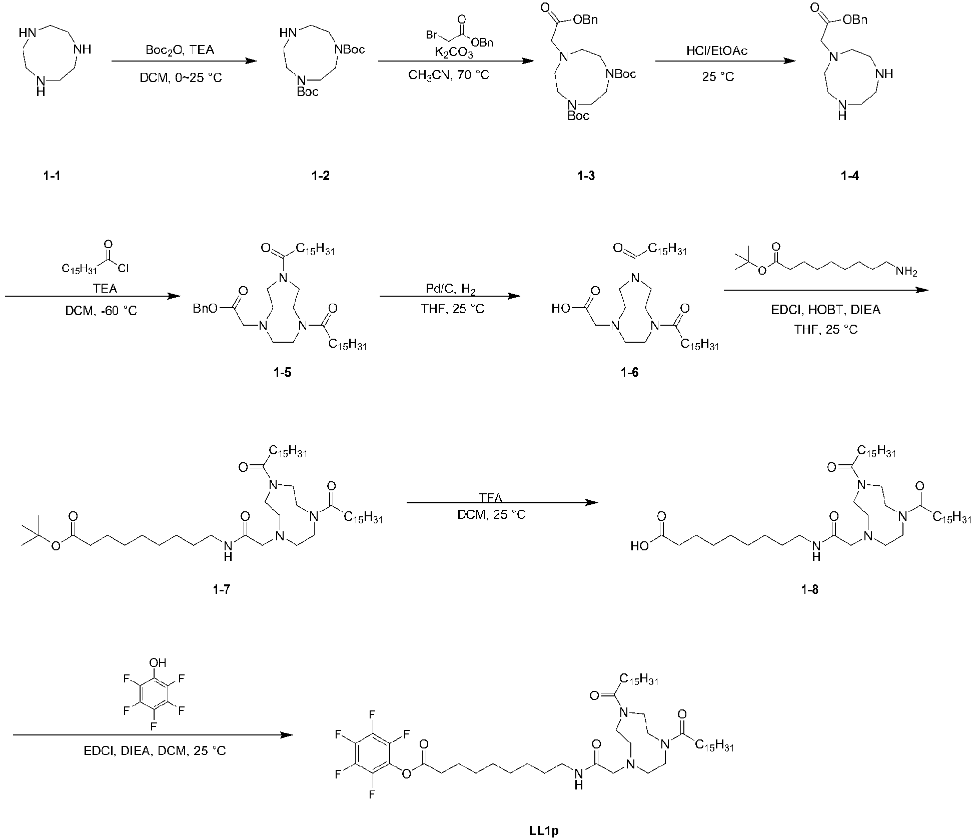

### Synthesis of Compound 1-2

At 25 °C, 1,4,7-triazacyclononane (50.00 g, 386.99 mmol) was dissolved in dichloromethane (500 mL), triethylamine (97.90 g, 967.47 mmol) was added, and di-tert-butyl dicarbonate (160.47 g, 735.28 mmol) was slowly added dropwise to the mixture at 0 °C, then stirred at 25 °C for 12 h. After completion of the reaction, water (1000 mL) was added to dilute and the mixture was extracted with dichloromethane (300 mL × 3). The combined organic phases were washed with saturated brine, dried over anhydrous sodium sulfate, filtered, and concentrated. The residue was purified by flash silica gel chromatography (eluent: 0~25% ethyl acetate/petroleum ether) to give Compound **1-2** as a white solid (50.00 g, 39.22% yield). ¹H NMR (400 MHz, CDCl₃) δ 3.54 - 3.40 (m, 4H), 3.35 - 3.15 (m, 4H), 2.93 (d, *J =* 4.9 Hz, 4H), 1.48 (s, 17H).

### Synthesis of Compound 1-3

At 25 °C, Compound **1-2** (50.00 g, 151.78 mmol) and benzyl bromoacetate (38.24 g, 166.95 mmol) were dissolved in acetonitrile (500 mL), potassium carbonate (41.95 g, 303.55 mmol) was added, and the mixture was stirred at 70 °C for 16 h. After completion of the reaction, water (300 mL) was added to dilute and the mixture was extracted with dichloromethane (300 mL × 3). The combined organic phases were washed with saturated brine, dried over anhydrous sodium sulfate, filtered, and concentrated. The residue was purified by flash silica gel chromatography (eluent: 0~25% ethyl acetate/petroleum ether) to give Compound **1-3** as a white solid (70.00 g, 96.57% yield). MS *m*/*z* (ESI): 478.3 [M+H]⁺; ¹H NMR (400 MHz, CDCl₃) δ 7.44 - 7.31 (m, 5H), 5.24 - 5.10 (m, 2H), 3.56 - 3.40 (m, 6H), 3.35 - 3.17 (m, 4H), 2.87 (dd, *J =* 5.5, 10.8 Hz, 4H), 1.48 (d, *J* = 4.5 Hz, 18H)*.*

### Synthesis of Compound 1-4

At 25 °C, Compound **1-3** (70.00 g, 146.57 mmol) was dissolved in dioxane (100 mL), hydrochloric acid/dioxane (2 M, 500 mL) was added, and the mixture was stirred at 25 °C for 16 h. After completion of the reaction, the organic phase was concentrated to give Compound **1-4** as a white solid (40.00 g, crude). MS *m*/*z* (ESI): 278.2 [M+H]⁺.

### Synthesis of Compound 1-5

At 25 °C, Compound **1-4** (40.00 g, 144.22 mmol) was dissolved in dichloromethane (500 mL), triethylamine (87.56 g, 865.30 mmol) was added, and palmitoyl chloride (79.28 g, 288.43 mmol) was slowly added dropwise to the mixture at -60 °C, then stirred at -60 °C for 3 h. After completion of the reaction, water (500 mL) was added to dilute and the mixture was extracted with dichloromethane (300 mL × 3). The combined organic phases were washed with saturated brine, dried over anhydrous sodium sulfate, filtered, and concentrated. The residue was purified by flash silica gel chromatography (eluent: 0~10% ethyl acetate/petroleum ether) to give Compound **1-5** as a white solid (85.00 g, 78.15% yield). MS *m*/*z* (ESI): 754.7 [M+H]⁺; ¹H NMR (400 MHz, CDCl₃) δ 7.34 - 7.24 (m, 5H), 5.09 - 5.05 (m, 2H), 3.69 - 3.51 (m, 4H), 3.38 (s, 2H), 3.31 - 3.16 (m, 4H), 2.95 - 2.71 (m, 4H), 2.24 - 2.10 (m, 4H), 1.26 - 1.14 (m, 52H), 0.81 (t, *J =* 6.8 Hz, 6H).

### Synthesis of Compound 1-6

At 25 °C, Compound **1-5** (100.00 g, 144.22 mmol) was dissolved in tetrahydrofuran (1000 mL) in a hydrogenation flask, then purged with nitrogen three times, wet palladium hydroxide (9.31 g, 20% purity) was added, and the flask was purged with nitrogen three times and then with hydrogen three times to 15 psi, and stirred at 25 °C for 16 h. After completion of the reaction, the reaction mixture was filtered and concentrated. The residue was purified by trituration with methanol to give Compound **1-6** as a white solid (65.00 g, 73.82% yield). MS *m*/*z* (ESI): 664.6 [M+H]⁺; ¹H NMR (400 MHz, CDCl₃) δ 3.81 - 3.62 (m, 4H), 3.52 - 3.28 (m, 6H), 3.03 - 2.78 (m, 4H), 2.38 - 2.21 (m, 4H), 1.63 (s, 4H), 1.27 (m, 48H), 0.89 (t, *J =* 6.7 Hz, 6H).

### Synthesis of Compound 1-7

At 25 °C, Compound **1-6** (7.00 g, 10.54 mmol) and 1-hydroxybenzotriazole (2.85 g, 21.08 mmol) were dissolved in tetrahydrofuran (70 mL), tert-butyl 9-aminononanoate (2.90 g, 12.65 mmol), N,N-diisopropylethylamine (4.09 g, 31.62 mmol), and 1-(3-dimethylaminopropyl)-3-ethylcarbodiimide hydrochloride (4.04 g, 21.08 mmol) were added, and the mixture was stirred at 25 °C for 3.5 h. After completion of the reaction, water (30 mL) was added to dilute and the mixture was extracted with dichloromethane (10 mL × 3). The combined organic phases were washed with saturated brine, dried over anhydrous sodium sulfate, filtered, and concentrated. The residue was purified by reversed-phase HPLC (column: C18 150×40 mm; mobile phase: [water (ammonia v/v) - (acetonitrile-tetrahydrofuran 2/1)]; gradient: 90% to 100% B over 9 min) to give Compound 1-7 as a yellow oil (9.00 g, 97.53% yield). MS *m*/*z* (ESI): 875.8 [M+H]⁺; ¹H NMR (400 MHz, CDCl₃) δ 6.85 - 6.71 (m, 1H), 3.75 - 3.58 (m, 4H), 3.50 - 3.33 (m, 4H), 3.29 - 3.17 (m, 4H), 2.78 (d, *J* = 4.5 Hz, 4H), 2.35 - 2.27 (m, 4H), 2.21 (t, *J =* 7.5 Hz, 2H), 1.69 - 1.55 (m, 6H), 1.46 (s, 9H), 1.38 - 1.22 (m, 58H), 0.90 (t, *J =* 6.8 Hz, 6H).

### Synthesis of Compound 1-8

At 25 °C, Compound **1-7** (6.50 g, 7.43 mmol) was dissolved in dichloromethane (50 mL), trifluoroacetic acid (25 mL) was added, and the mixture was stirred at 25 °C for 3 h. After completion of the reaction, water (50 mL) was added to dilute and the mixture was extracted with dichloromethane (50 mL × 3). The combined organic phases were washed with saturated brine, dried over anhydrous sodium sulfate, filtered, and concentrated. The residue was purified by flash silica gel chromatography (eluent: 0~25% tetrahydrofuran/petroleum ether) to give Compound **1-8** as a white solid (5.50 g, 90.41% yield). MS *m*/*z* (ESI): 819.8 [M+H]⁺; ¹H NMR (400 MHz, CDCl₃) δ 7.44 - 7.31 (m, 5H), 5.24 - 5.10 (m, 2H), 3.56 - 3.40 (m, 6H), 3.35 - 3.17 (m, 4H), 2.87 (dd, *J* = 5.5, 10.8 Hz, 4H), 1.48 (d, *J* = 4.5 Hz, 18H).

### Synthesis of Compound LL1p

At 25 °C, Compound **1-8** (2.00 g, 2.44 mmol) was dissolved in dichloromethane (300 mL), 2,3,4,5,6-pentafluorophenol (539.19 mg, 2.93 mmol) was added, and the mixture was stirred at 25 °C for 3 h. After completion of the reaction, the organic phase was concentrated, and the residue was purified by flash silica gel chromatography (eluent: 0~20% tetrahydrofuran/petroleum ether) to give Compound **LL1p** as a colorless amorphous solid (1.20 g, 49.89% yield). MS *m*/*z* (ESI): 985.7 [M+H]⁺; ¹H NMR (400 MHz, CDCl₃) δ 6.78 - 6.62 (m, 1H), 3.67 - 3.50 (m, 4H), 3.42 - 3.24 (m, 4H), 3.21 - 3.09 (m, 4H), 2.76 - 2.64 (m, 4H), 2.59 (t, *J =* 7.4 Hz, 2H), 2.22 (q, *J =* 8.2 Hz, 4H), 1.77 - 1.32 (m, 12H), 1.27 - 1.13 (m, 52H), 0.81 (t, *J =* 6.8 Hz, 6H).

### Example 2. Synthesis of LL2p

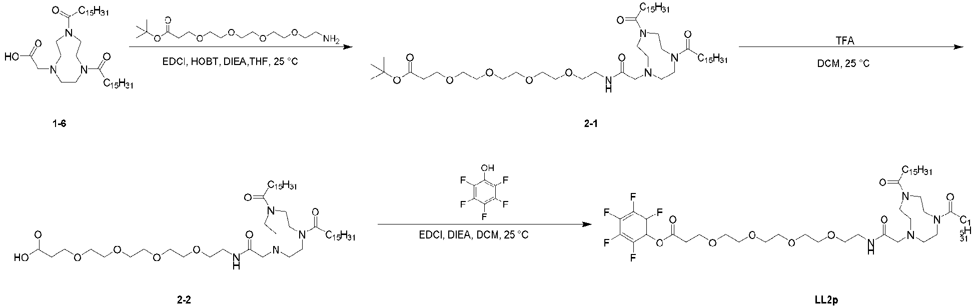

### Synthesis of Compound 2-1

At 25 °C, Compound **1-6** (7.00 g, 10.54 mmol) and 1-hydroxybenzotriazole (2.85 g, 21.08 mmol) were dissolved in tetrahydrofuran (70 mL), tert-butyl 15-amino-4,7,10,13-tetraoxapentadecanoate (3.73 g, 11.60 mmol), N,N-diisopropylethylamine (4.09 g, 31.62 mmol), and 1-(3-dimethylaminopropyl)-3-ethylcarbodiimide hydrochloride (4.04 g, 21.08 mmol) were added, and the mixture was stirred at 25 °C for 3.5 h. After completion of the reaction, water (30 mL) was added to dilute and the mixture was extracted with dichloromethane (10 mL × 3). The combined organic phases were washed with saturated brine, dried over anhydrous sodium sulfate, filtered, and concentrated. The residue was purified by reversed-phase HPLC (column: C18 150×40 mm; mobile phase: [water (ammonia v/v) - (acetonitrile-tetrahydrofuran 2/1)]; gradient: 85% to 100% B over 9 min) to give Compound **2-1** as a yellow oil (10.00 g, 98.06% yield). MS *m*/*z* (ESI): 967.8 [M+H]⁺; ¹H NMR (400 MHz, CDCl₃) δ 7.25 - 7.06 (m, 1H), 3.71 (d, *J =* 7.8 Hz, 4H), 3.65 - 3.62 (m, 10H), 3.57 (t, *J =* 5.0 Hz, 2H), 3.47 (d, *J =* 5.0 Hz, 6H), 3.26 - 3.18 (m, 2H), 2.88 - 2.73 (m, 4H), 2.52 (t, *J* = 6.5 Hz, 2H), 2.38 - 2.23 (m, 4H), 1.78 (s, 4H), 1.70 - 1.58 (m, 4H), 1.46 (s, 9H), 1.34 - 1.24 (m, 48H), 0.90 (t, *J =* 6.7 Hz, 6H).

### Synthesis of Compound 2-2

At 25 °C, Compound **2-1** (7.00 g, 7.24 mmol) was dissolved in dichloromethane (50 mL), trifluoroacetic acid (25 mL) was added, and the mixture was stirred at 25 °C for 3 h. After completion of the reaction, water (50 mL) was added to dilute and the mixture was extracted with dichloromethane (50 mL × 3). The combined organic phases were washed with saturated brine, dried over anhydrous sodium sulfate, filtered, and concentrated. The residue was purified by flash silica gel chromatography (eluent: 0~25% tetrahydrofuran/petroleum ether) to give Compound 2-2 as a white solid (6.50 g, 98.57% yield). MS *m*/*z* (ESI): 911.8 [M+H]⁺; ¹H NMR (400 MHz, CDCl₃) δ 7.69 - 7.33 (m, 1H), 4.40 - 3.21 (m, 20H), 2.49 - 2.26 (m, 6H), 1.52 (s, 2H), 1.64 (s, 5H), 1.40 - 1.22 (m, 56H), 0.90 (t, *J =* 6.7 Hz, 6H).

### Synthesis of Compound LL2p

At 25 °C, Compound **2-2** (2.00 g, 2.44 mmol) was dissolved in dichloromethane (30 mL), 2,3,4,5,6-pentafluorophenol (539.19 mg, 2.93 mmol) was added, and the mixture was stirred at 25 °C for 3 h. After completion of the reaction, the organic phase was concentrated, and the residue was purified by flash silica gel chromatography (eluent: 0~20% tetrahydrofuran/petroleum ether) to give Compound **LL2p** as a colorless amorphous solid (1.20 g, 50.75% yield). MS *m*/*z* (ESI): 1077.7 [M+H]⁺; ¹H NMR (400 MHz, CDCl₃) δ 7.24 - 6.99 (m, 1H), 3.89 (t, *J =* 6.2 Hz, 2H), 3.74 - 3.61 (m, 16H), 3.60 - 3.55 (m, 2H), 3.51 - 3.32 (m, 6H), 3.23 (s, 2H), 2.96 (t, *J =* 6.2 Hz, 2H), 2.87 - 2.73 (m, 4H), 2.36 - 2.24 (m, 4H), 1.71 - 1.57 (m, 4H), 1.35 - 1.24 (m, 48H), 0.97 - 0.82 (m, 6H).

### Example 3. Synthesis of Lipid-Oligonucleotide Conjugates

Synthesis of Oligonucleotides

The synthesis of oligonucleotides followed conventional phosphoramidite solid-phase synthesis methods (all entrusted to Suzhou Beixin Biotechnology Co., Ltd.), including four steps: deprotection, coupling, capping, and oxidation or sulfurization. Starting with the solid support, nucleoside monomers were sequentially linked in the 3' to 5' order. Nucleoside phosphoramidite monomers such as 2'-F RNA, 2'-OMe RNA, etc. were purchased from Shanghai Zhaowei Science & Technology Co., Ltd. After solid-phase synthesis, the solid support was transferred to a centrifuge tube, and a 3:1 mixture of 28% ammonia water and ethanol solution was added to soak the support at 50 °C for 16 h to cleave the oligonucleotides from the support into solution. After centrifugation, the supernatant was transferred to another centrifuge tube, concentrated to dryness, redissolved in deionized water, and purified using a C18 reversed-phase column with mobile phases of 0.1 M TEAA and acetonitrile. The target oligonucleotide fractions were collected, lyophilized, identified as the desired product by LC-MS, and quantified by UV (260 nm). The resulting sense and antisense strands were annealed at a molar ratio to form complementary paired double-stranded siRNA and adjusted to the desired concentration.

### Synthesis of Lipid-Oligonucleotide Conjugates

The synthesized oligonucleotide with an -NH2 group (1.0 equiv.) was dissolved in DMSO, and **LL1p** or **LL2p** compound (15 equiv.) dissolved in DMSO was added, followed by addition of triethylamine. The mixture was vortexed at 25 °C for 2 h. After the reaction was complete as monitored by LCMS, the product was purified by reversed-phase HPLC to obtain the desired oligonucleotide. The target oligonucleotide fractions were collected, lyophilized, identified by LC-MS, and quantified by UV (260 nm). The resulting sense and antisense strands were annealed at a molar ratio to form complementary paired double-stranded siRNA and adjusted to the desired concentration. Exemplary lipid-oligonucleotide conjugates synthesized are shown in Table 1.

**Table 1. Lipid-siRNA conjugates targeting superoxide dismutase 1 (SOD1)**

| Duplex | | (5'-3') | Calc MW | Found MW | SEQ ID NO. |
|---|---|---|---|---|---|
| **D0** | SS | NH2C6-mC*mA*mU mUmUmU fAmAfU fCfCmU mCmAmC mUmCmU mA*mA*mA | 7012.7 | 7013.6 | 1 |
| | AS | VPmU*fU*mU mAmGfA mGfUfG mAmGmG mAfUmU fAmAmA mAmUmG* mA*mG | 7851.2 | 7852.5 | 2 |
| **D1** | SS | LL1-NHC6-mC*mA*mU mUmUmU fAmAfU fCfCmU mCmAmC mUmCmU mA*mA*mA | 7814.1 | 7815.0 | 3 |
| | AS | VPmU*fU*mU mAmGfA mGfUfG mAmGmG mAfUmU fAmAmA mAmUmG* mA*mG | 7851.2 | 7852.5 | 2 |
| **D2** | SS | LL2-NHC6-mC*mA*mU mUmUmU fAmAfU fCfCmU mCmAmC mUmCmU mA*mA*mA | 7906.1 | 7907.7 | 4 |
| | AS | VPmU*fU*mU mAmGfA mGfUfG mAmGmG mAfUmU fAmAmA mAmUmG* mA*mG | 7851.2 | 7852.5 | 2 |

wherein m represents 2'-O-methyl modification, f represents 2'-fluoro modification, * represents phosphorothioate modification, SS represents sense strand, and AS represents antisense strand.

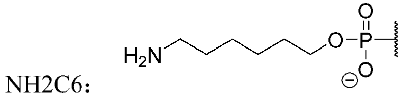

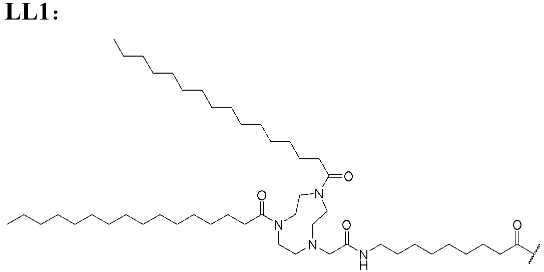

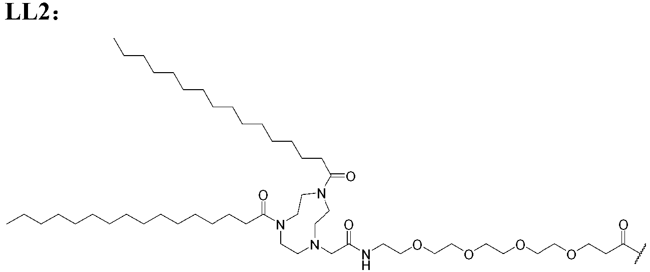

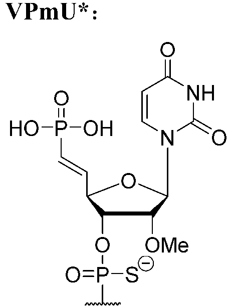

### Example 4. In Vivo Efficacy Evaluation of Lipid-Oligonucleotide Conjugates in Mice

Male C57BL/6 mice, 6-8 weeks old (N=6 per group), were intravenously injected with a single dose of 2 mg/kg or 10 mg/kg of lipid-siRNA conjugates **D1** and **D2.**

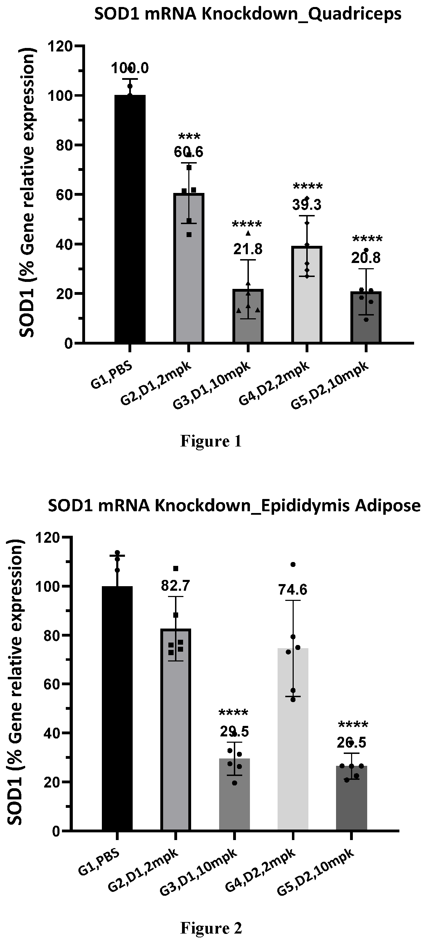

On day 14 post-administration, quadriceps and epididymal adipose tissues were collected, immediately snap-frozen in liquid nitrogen, and stored at -80 °C. RNA was extracted and purified from tissue samples using a silica-based adsorption column method, and the resulting RNA was used for cDNA synthesis with a reverse transcription kit (TAKARA, RR047A). SOD1 mRNA levels were quantified by RT-qPCR using a Bio-Rad CFX96. The relative change in SOD1 mRNA expression was determined using the ΔΔCt method with double normalization to GAPDH and to the mean of the control group. Results are shown in Figures 1 and 2, indicating that lipid-siRNA conjugates **D1** and **D2** both exhibited dose-dependent inhibition of SOD1 mRNA expression in quadriceps (Figure 1) and epididymal adipose tissue (Figure 2).

## Claims

1. A lipid-oligonucleotide conjugate comprising a structure of Formula Ia: 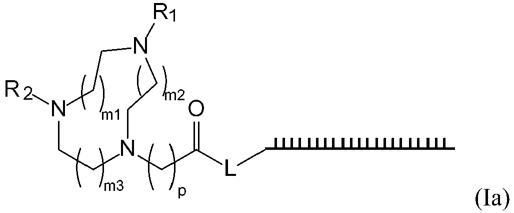 wherein,
at least one of R1 and R2 is selected from Ra, -C(O)Ra, -C(O)NHRa, and C(O)ORa, wherein Ra is optionally substituted C5-30 alkyl or alkenyl, and
the other of R1 and R2 is selected from H, Rb, -C(O)Rb, -C(O)NHRb, and C(O)ORb, wherein Rb is selected from optionally substituted C1-4 alkyl or alkenyl, optionally substituted C5-30 alkyl or alkenyl, optionally substituted C2-10 alkynyl, optionally substituted C1-10 alkoxy, optionally substituted C3-10 cycloalkyl, optionally substituted 6- to 14-membered aryl, optionally substituted 5- to 18-membered heteroaryl, and optionally substituted 5- to 14-membered heterocyclyl;
said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5- to 18-membered heteroaryl, 5- to 14-membered heterocyclyl, halogen, hydroxyl, amino, guanidino, carboxyl, cyano, nitro, C1-3 alkylthio, and mercapto;
m1, m2, and m3 are each independently an integer of 1 or 2;
p is 0, 1, or 2; 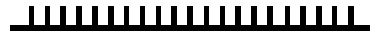
represents an oligonucleotide;
L represents a linker.

2. The lipid-oligonucleotide conjugate according to claim 1, wherein at least one or both of R1 and R2 are independently -C(O)Ra, wherein Ra is optionally substituted C5-30 alkyl or alkenyl;
preferably, Ra is optionally substituted C12-24 alkyl or alkenyl;
preferably, Ra is optionally substituted C14-22 alkyl or alkenyl;
more preferably, Ra is optionally substituted C14, C15, C16, C18, C20, or C22 alkyl, monounsaturated alkenyl, or polyunsaturated alkenyl.

3. The lipid-oligonucleotide conjugate according to claim 1 or 2, wherein m1, m2, and m3 are all 1, or two of m1, m2, and m3 are 1 and the other is 2.

4. The lipid-oligonucleotide conjugate according to any one of claims 1 to 3, wherein p is 1.

5. The lipid-oligonucleotide conjugate according to any one of claims 1 to 4, wherein the oligonucleotide is an antisense oligonucleotide or the sense strand or antisense strand of an RNAi agent;
preferably, the 5' and/or 3' end of said oligonucleotide is linked to L via a phosphate group, phosphorothioate group, or phosphonate group;
preferably, the sense strand or antisense strand of said RNAi agent pairs with its antisense strand or sense strand to form a duplex;
preferably, said RNAi agent is siRNA;
preferably, said oligonucleotide is the sense strand of siRNA;
preferably, the antisense strand of said siRNA has a 5'-VP modification.

6. The lipid-oligonucleotide conjugate according to any one of claims 1 to 5, wherein:
L is -NH-(CH2)q-O-, -NH-(CH2CH2O)q-, 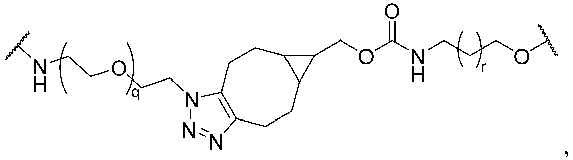 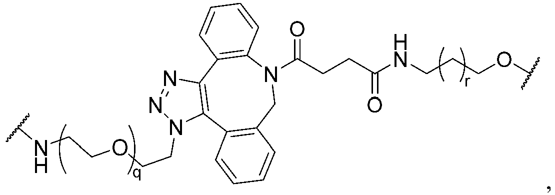 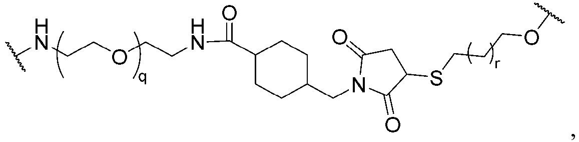 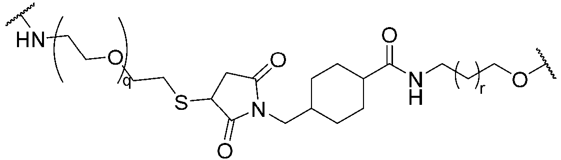 or 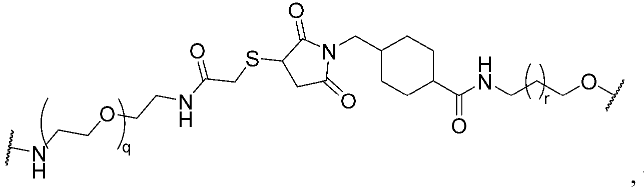 wherein q and r are independently integers from 1 to 20, and the right side of the formula is conjugated to the oligonucleotide; or
L comprises L1-L2, wherein L1 is 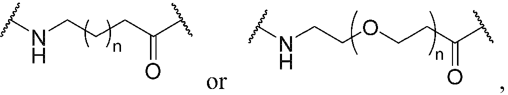 wherein the right side of the formula is connected to L2; L2 is 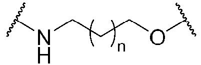 or 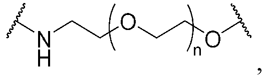 wherein the left side of the formula is connected to L1; the wavy line represents the connection position; and wherein each n is independently an integer from 1 to 10, preferably from 2 to 8, more preferably from 4 to 6.

7. A lipid-oligonucleotide conjugate represented by Formula Ib, 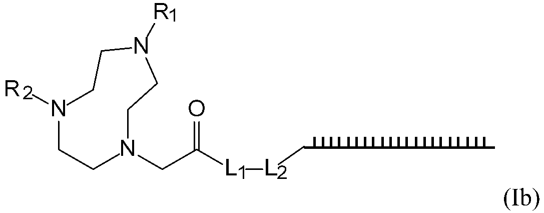 wherein,
at least one of R1 and R2 is selected from Ra, -C(O)Ra, -C(O)NHRa, and C(O)ORa, wherein Ra is optionally substituted C5-30 alkyl or alkenyl, and
the other of R1 and R2 is selected from H, Rb, -C(O)Rb, -C(O)NHRb, and C(O)ORb, wherein Rb is selected from optionally substituted C1-4 alkyl or alkenyl, optionally substituted C5-30 alkyl or alkenyl, optionally substituted C2-10 alkynyl, optionally substituted C1-10 alkoxy, optionally substituted C3-10 cycloalkyl, optionally substituted 6- to 14-membered aryl, optionally substituted 5- to 18-membered heteroaryl, optionally substituted 5- to 14-membered heterocyclyl;
said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5- to 18-membered heteroaryl, 5- to 14-membered heterocyclyl, halogen, hydroxyl, amino, guanidino, carboxyl, cyano, nitro, C1-3 alkylthio, and mercapto;
L1 is 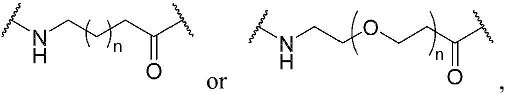 wherein the right side of the formula is connected to L2; L2 is 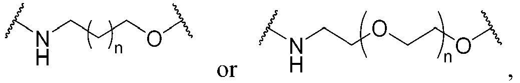 wherein the left side of the formula is connected to L1; the wavy line represents the connection position; and wherein each n is independently an integer from 1 to 10, preferably from 2 to 8, more preferably from 4 to 6; 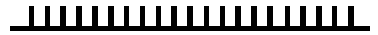
represents an oligonucleotide, preferably the oligonucleotide is an antisense oligonucleotide or the sense strand or antisense strand of an RNAi agent; preferably the 5' and/or 3' end of said oligonucleotide is linked to L2 via a phosphate group, phosphorothioate group, or phosphonate group; preferably the sense strand or antisense strand of said RNAi agent pairs with its antisense strand or sense strand to form a duplex; preferably said RNAi agent is siRNA; preferably said oligonucleotide is the sense strand of siRNA; preferably the antisense strand of said siRNA has a 5'-VP modification.

8. A lipid-oligonucleotide conjugate represented by Formula Ic, 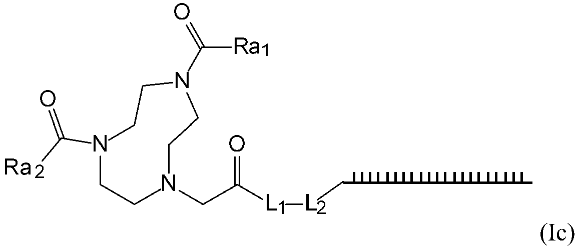 wherein,
at least one of Ra1 and Ra2 is optionally substituted C5-30 alkyl or alkenyl, preferably optionally substituted C12-24 alkyl or alkenyl; preferably optionally substituted C14-22 alkyl or alkenyl; more preferably optionally substituted C14, C15, C16, C18, C20, or C22 alkyl, monounsaturated alkenyl, or polyunsaturated alkenyl;
the other of Ra1 and Ra2 is optionally substituted C1-4 alkyl or alkenyl, optionally substituted C5-30 alkyl or alkenyl, optionally substituted C2-10 alkynyl, optionally substituted C1-10 alkoxy, optionally substituted C3-10 cycloalkyl, optionally substituted 6- to 14-membered aryl, optionally substituted 5- to 18-membered heteroaryl, or optionally substituted 5- to 14-membered heterocyclyl;
said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5- to 18-membered heteroaryl, 5- to 14-membered heterocyclyl, halogen, hydroxyl, amino, guanidino, carboxyl, cyano, nitro, C1-3 alkylthio, and mercapto;
L1 is 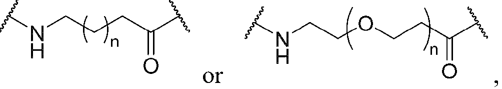 wherein the right side of the formula is connected to L2; L2 is 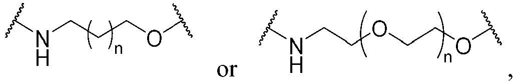 wherein the left side of the formula is connected to L1; the wavy line represents the connection position; and wherein each n is independently an integer from 1 to 10, preferably from 2 to 8, more preferably from 4 to 6; 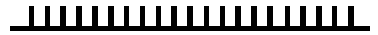
represents an oligonucleotide, preferably the oligonucleotide is an antisense oligonucleotide or the sense strand or antisense strand of an RNAi agent; preferably the 5' and/or 3' end of said oligonucleotide is linked to L2 via a phosphate group, phosphorothioate group, or phosphonate group; preferably the sense strand or antisense strand of said RNAi agent pairs with its antisense strand or sense strand to form a duplex; preferably said RNAi agent is siRNA; preferably said oligonucleotide is the sense strand of siRNA; preferably the antisense strand of said siRNA has a 5'-VP modification.

9. A lipid-oligonucleotide conjugate selected from the following structural formulae: 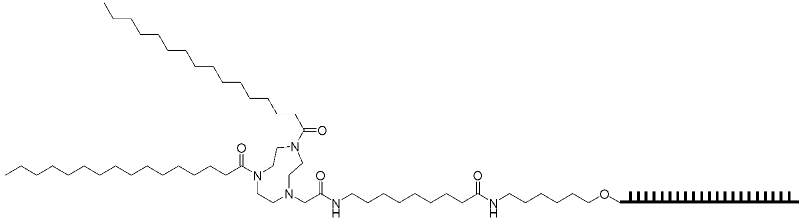 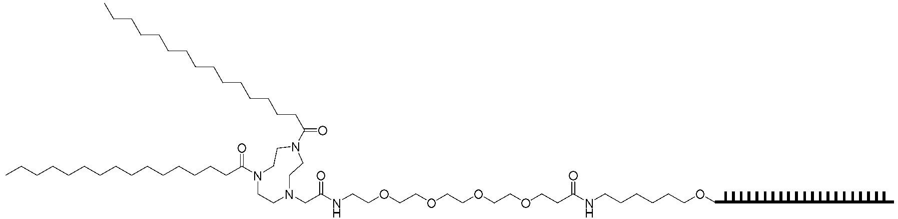 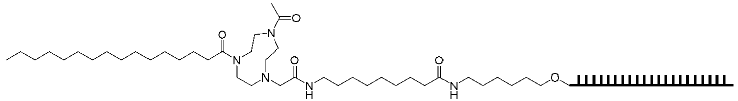 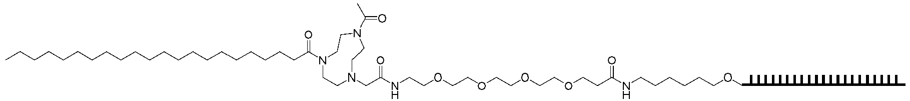 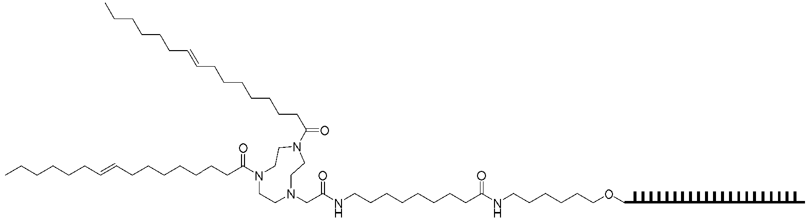 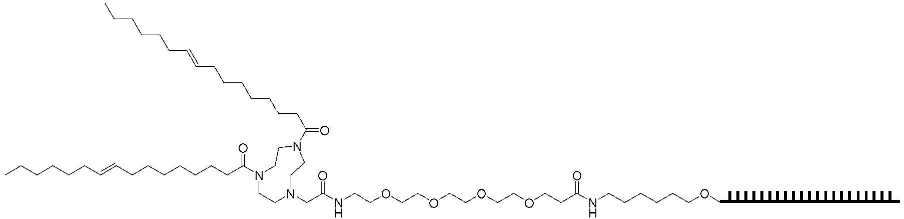 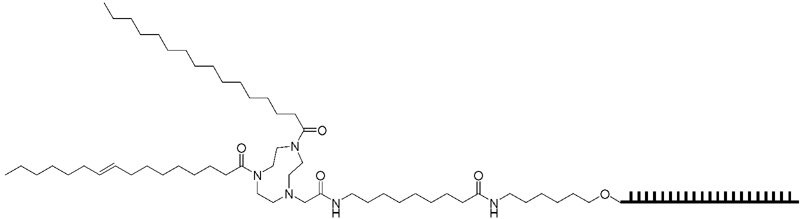 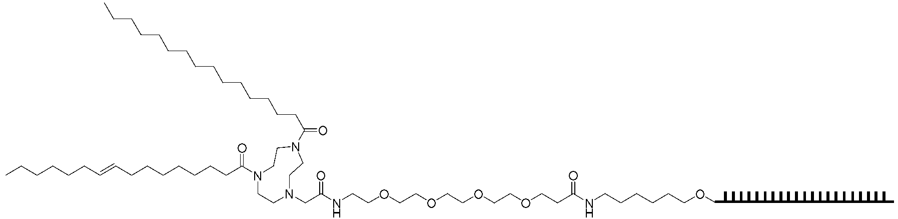 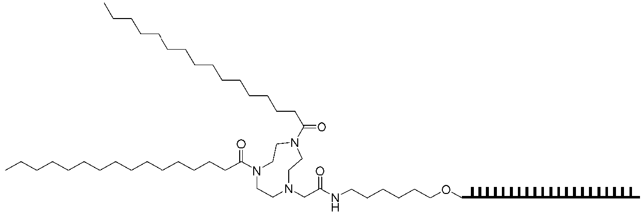 wherein 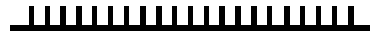 represents an oligonucleotide, preferably the oligonucleotide is an antisense oligonucleotide or the sense strand or antisense strand of an RNAi agent; preferably the 5' and/or 3' end of said oligonucleotide is conjugated via a phosphate group, phosphorothioate group, or phosphonate group; preferably the sense strand or antisense strand of said RNAi agent pairs with its antisense strand or sense strand to form a duplex; preferably said RNAi agent is siRNA; preferably said oligonucleotide is the sense strand of siRNA; preferably the antisense strand of said siRNA has a 5'-VP modification.

10. The lipid-oligonucleotide conjugate according to claim 9, selected from any one of the following structures: 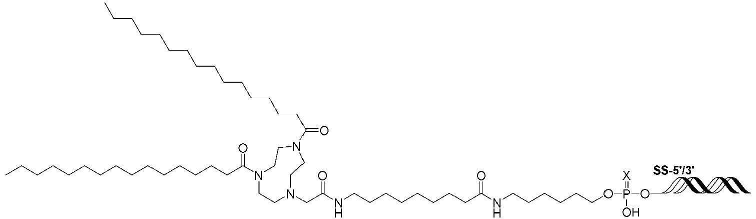 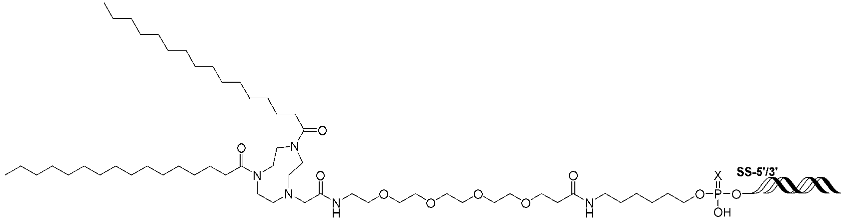 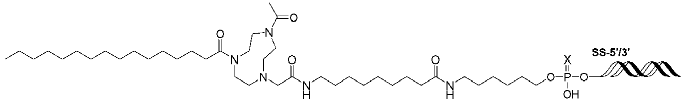 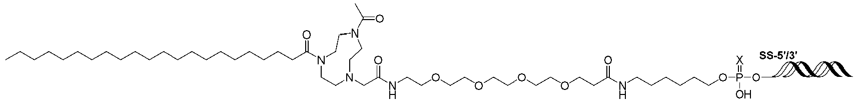 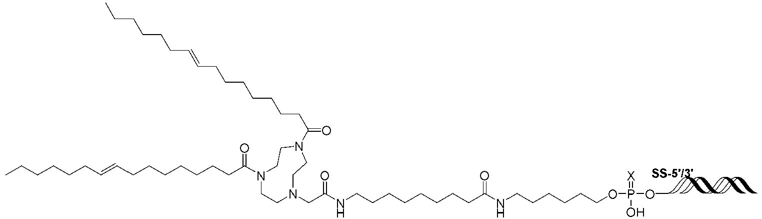 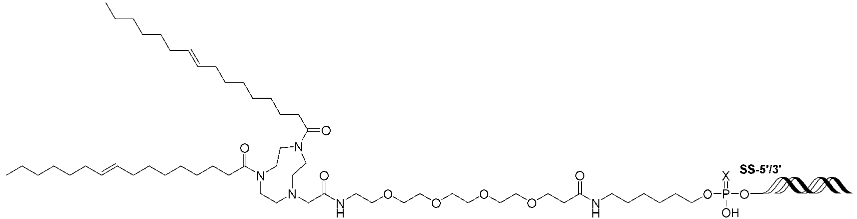 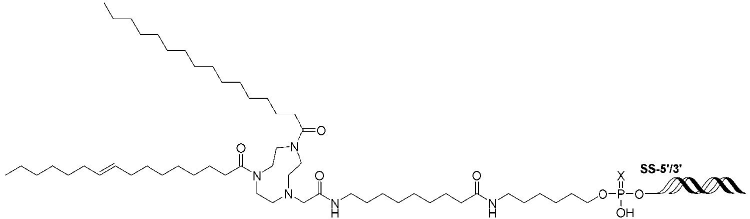 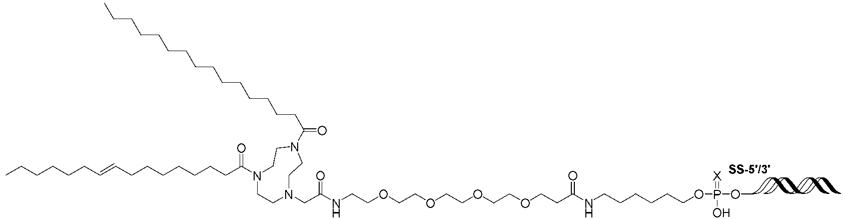 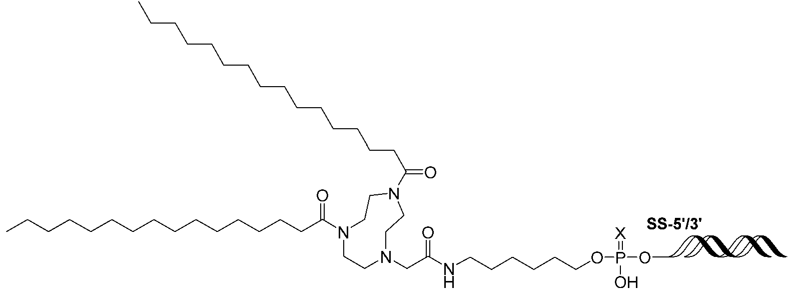 wherein, 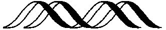 represents siRNA, X is S or O, and SS-5'/3' represents the 5' or 3' end of the sense strand.

11. A compound selected from the following structural formulae:
| | |
|---|---|
| **1-5** | 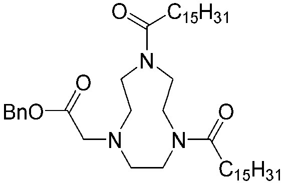 |
| **1-6** | 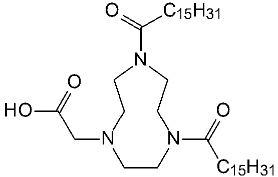 |
| **1-7** | 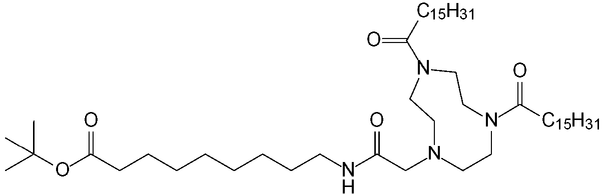 |
| **1-8** | 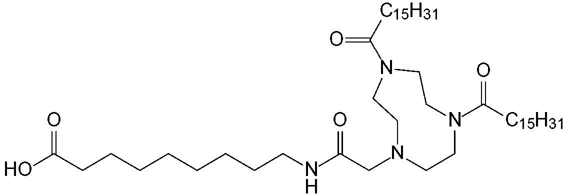 |
| **LL1p** | 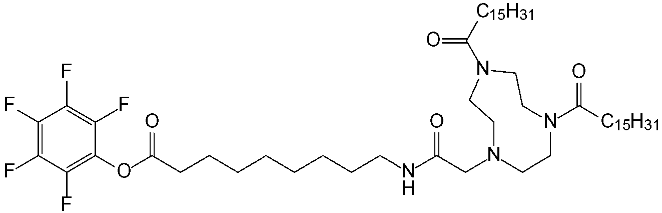 |
| **2-1** | 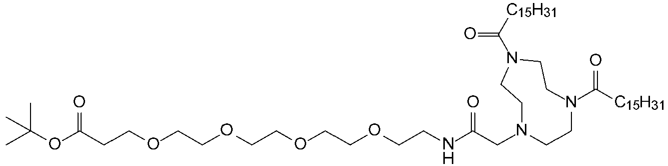 |
| **2-2** | 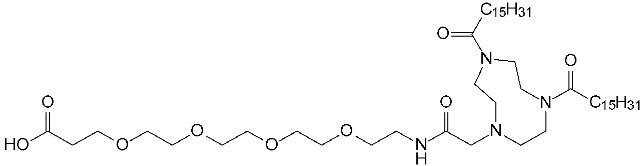 |
| **LL2p** | 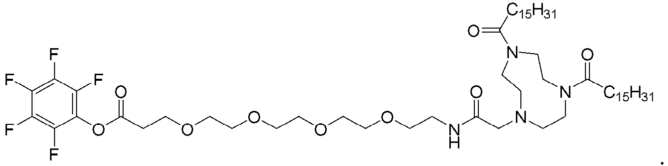 |

12. A ligand moiety represented by Formula II for forming a lipid-oligonucleotide conjugate: 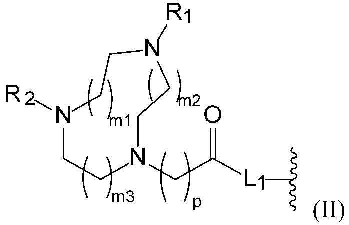 wherein,
at least one of R1 and R2 is selected from Ra, -C(O)Ra, -C(O)NHRa, and C(O)ORa, wherein Ra is optionally substituted C5-30 alkyl or alkenyl, and
the other of R1 and R2 is selected from H, Rb, -C(O)Rb, -C(O)NHRb, and C(O)ORb, wherein Rb is selected from optionally substituted C1-4 alkyl or alkenyl, optionally substituted C5-30 alkyl or alkenyl, optionally substituted C2-10 alkynyl, optionally substituted C1-10 alkoxy, optionally substituted C3-10 cycloalkyl, optionally substituted 6- to 14-membered aryl, optionally substituted 5- to 18-membered heteroaryl, optionally substituted 5- to 14-membered heterocyclyl;
said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5- to 18-membered heteroaryl, 5- to 14-membered heterocyclyl, halogen, hydroxyl, amino, guanidino, carboxyl, cyano, nitro, C1-3 alkylthio, and mercapto;
m1, m2, and m3 are each independently an integer of 1 or 2;
p is 0, 1, or 2; and 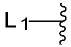
represents a linker, and the wavy line represents the position of attachment to the remainder of the lipid-oligonucleotide conjugate.

13. The ligand moiety according to claim 12, wherein 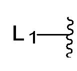 is 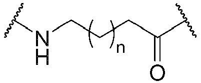 or 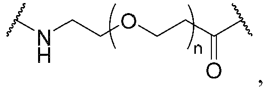 wherein each n is independently an integer from 1 to 10, preferably from 2 to 8, more preferably from 4 to 6; and the right side of the formula is connected to the remainder of the lipid-oligonucleotide conjugate.

14. The ligand moiety according to claim 12 or 13, having one or more of the following features:
(a) at least one or both of R1 and R2 are independently -C(O)Ra, wherein Ra is optionally substituted C5-30 alkyl or alkenyl; preferably, Ra is optionally substituted C12-24 alkyl or alkenyl; preferably, Ra is optionally substituted C14-22 alkyl or alkenyl; more preferably, Ra is optionally substituted C14, C15, C16, C18, C20, or C22 alkyl, monounsaturated alkenyl, or polyunsaturated alkenyl;
(b) m1, m2, and m3 are all 1, or two of m1, m2, and m3 are 1 and the other is 2;
(c) p is 1;
(d) the oligonucleotide is an antisense oligonucleotide or the sense strand or antisense strand of an RNAi agent; preferably the sense strand or antisense strand of said RNAi agent pairs with its antisense strand or sense strand to form a duplex; preferably said RNAi agent is siRNA; preferably said oligonucleotide is the sense strand of siRNA; preferably the antisense strand of said siRNA has a 5'-VP modification.

15. The ligand moiety according to claim 12, which is 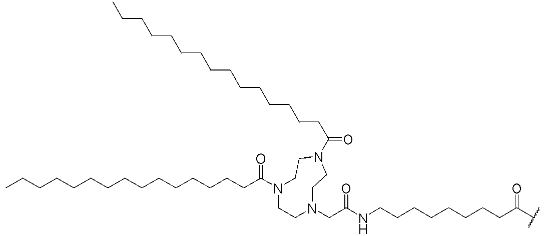 or

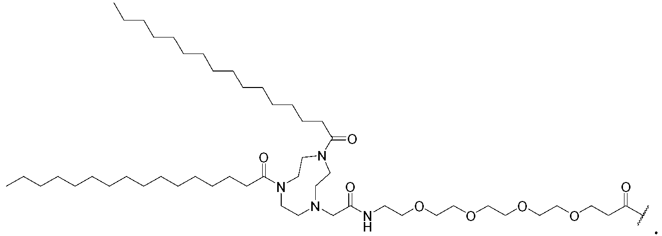

16. Use of a ligand moiety represented by Formula III in the preparation of a lipid-oligonucleotide conjugate, 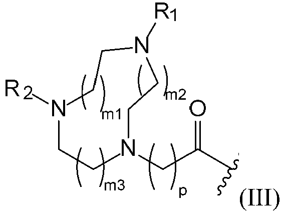 wherein,
at least one of R1 and R2 is selected from Ra, -C(O)Ra, -C(O)NHRa, and C(O)ORa, wherein Ra is optionally substituted C5-30 alkyl or alkenyl, and
the other of R1 and R2 is selected from H, Rb, -C(O)Rb, -C(O)NHRb, and C(O)ORb, wherein Rb is selected from optionally substituted C1-4 alkyl or alkenyl, optionally substituted C5-30 alkyl or alkenyl, optionally substituted C2-10 alkynyl, optionally substituted C1-10 alkoxy, optionally substituted C3-10 cycloalkyl, optionally substituted 6- to 14-membered aryl, optionally substituted 5- to 18-membered heteroaryl, optionally substituted 5- to 14-membered heterocyclyl;
said optionally substituted is optionally substituted with Rc, wherein Rc is selected from C1-10 alkyl, C2-10 alkenyl, C2-10 alkynyl, C1-10 alkoxy, C3-10 cycloalkyl, 6- to 14-membered aryl, 5- to 18-membered heteroaryl, 5- to 14-membered heterocyclyl, halogen, hydroxyl, amino, guanidino, carboxyl, cyano, nitro, C1-3 alkylthio, and mercapto;
m1, m2, and m3 are each independently an integer of 1 or 2;
p is 0, 1, or 2; and
the wavy line represents the position of attachment to the remainder of the lipid-oligonucleotide conjugate.

17. The use according to claim 16, having one or more of the following features:
(a) at least one or both of R1 and R2 are independently -C(O)Ra, wherein Ra is optionally substituted C5-30 alkyl or alkenyl; preferably, Ra is optionally substituted C12-24 alkyl or alkenyl; preferably, Ra is optionally substituted C14-22 alkyl or alkenyl; more preferably, Ra is optionally substituted C14, C15, C16, C18, C20, or C22 alkyl, monounsaturated alkenyl, or polyunsaturated alkenyl;
(b) m1, m2, and m3 are all 1, or two of m1, m2, and m3 are 1 and the other is 2;
(c) p is 1;
(d) the oligonucleotide is an antisense oligonucleotide or the sense strand or antisense strand of an RNAi agent; preferably the sense strand or antisense strand of said RNAi agent pairs with its antisense strand or sense strand to form a duplex; preferably said RNAi agent is siRNA; preferably said oligonucleotide is the sense strand of siRNA; preferably the antisense strand of said siRNA has a 5'-VP modification.

18. A pharmaceutical composition comprising the lipid-oligonucleotide conjugate according to any one of claims 1 to 10; and a pharmaceutically acceptable carrier; preferably, the pharmaceutical composition is formulated as an intravenous or subcutaneous injection.

19. Use of the lipid-oligonucleotide conjugate according to any one of claims 1 to 10, the compound of claim 11, or the ligand moiety according to any one of claims 12 to 15 in the preparation of a lipid-oligonucleotide conjugate.
